# EUROPEAN PATENT APPLICATION

(11) **EP 4 744 625 A2**
(43) Date of publication of application: **20.05.2026**
(21) Application number: 26169270.1
(22) Date of filing: 20.05.2022
(51) Int. Cl.: A61F 2/24

(54) **COMMISSURAL ALIGNMENT SYSTEM AND METHOD OF ALIGNMENT THEREOF FOR PROSTHETIC VALVES**

(30) Priority: 12.10.2021 IN 202121046556
(62) Divisional of application: 22817052.8
(71) Applicant: Meril Life Sciences Pvt Ltd, Gujarat, Vapi 396191 (IN)
(72) Inventor: BHATT, Sanjeev Nauttam, 400064 Mumbai (IN); PARMAR, Harshad Amrutlal, 396191 Vapi (IN)
(74) Representative: Marks & Clerk LLP

(57) **Abstract**

A method for implanting a prosthetic valve in a patient's body with minimum misalignment of commissures of the prosthetic valve to the commissures of a native aortic valve is disclosed. AoCA of a native aortic valve of a patient to be treated is determined and the prosthetic valve having three commissures is crimped on a delivery system having one or more aligners marked on its outer shaft. The aligners follow same axis. Crimping is done using a crimper and/or a confirmation gauge that includes one or more angle markings. Crimping is performed such that one of the commissures of the prosthetic valve is axially aligned with the AoCA identified on the angle markings on one of the crimper/confirmation gauge and at the same time, the one or more aligners face upwards. The crimped prosthetic valve is implanted by maintaining the aligner(s) pointing upward during the entire implantation procedure.

## Description

### FIELD OF INVENTION

The present invention relates to a system. More specifically, the present invention relates to a system and method of achieving minimum misalignment of commissures of a trans-catheter prosthetic heart valve with commissures of a native heart valve.

### BACKGROUND

The function of a prosthetic heart valve is to replace a diseased native heart valve. The replacement procedure may be surgical (using open heart surgery) or percutaneous.

In a surgical procedure, the leaflets of the diseased native heart valve are excised and the annulus is sculpted to receive a prosthetic heart valve. For many years, the definitive treatment for such disorders was the surgical repair or replacement of the native heart valve during open heart surgery, but such surgeries are prone to many complications. Some patients do not survive the surgical procedure due to the trauma associated with the procedure and duration of extracorporeal blood circulation. Due to this, a number of patients are deemed inoperable and hence remain untreated.

Against the surgical procedure, a percutaneous catheterization technique has been developed for introducing and implanting a prosthetic heart valve using a flexible catheter that is considerably less invasive than an open heart surgery. In this technique, a prosthetic valve is mounted by crimping on a balloon located at the distal end of a flexible catheter, termed as trans-catheter heart valve system (THV). The catheter is most commonly introduced into a blood vessel usually through a peripheral artery (rarely via a vein); most likely a common femoral or sometimes axillary, carotid or subclavian artery of the patient or rarely via transapical route (through the apex of the heart) or transcaval route (through the vein and crossed over into the aorta) amongst other access routes. The catheter with the prosthetic valve crimped on the balloon is then advanced through the blood vessel till the crimped valve reaches the implantation site. The valve is allowed to expand to its functional size at the site of the defective native valve by inflating the balloon on which the valve has been mounted. Alternatively, the valve may have a self-expanding stent or support frame that expands the valve to its functional size by withdrawing a restraining sheath (retaining sheath) mounted over the prosthetic valve. The former prosthetic valve is termed as "balloon expandable" and the latter as "self-expanding".

Transcatheter aortic valve replacement (TAVR) has become a promising therapy in cases of symptomatic, severe aortic valve stenosis over a surgical aortic valve replacement (SAVR).

A very important unmet clinical need exists for a trans-catheter heart valve system (THV) viz. Commissural Alignment which includes aligning the commissures of a prosthetic heart valve with the commissures of the native aortic heart valve that is being treated. Commissural alignment (CA) of a THV is clinically important for several reasons outlined below. In real world TAVR procedures, CA is not routinely practiced as there is no simple method to achieve CA. Hence, CA is an important unmet clinical need.

The present invention is targeted to address the aforesaid unmet need.

### SUMMARY

The present invention relates to a system and method to achieve commissural alignment (CA) i.e. positioning of a prosthetic heart valve (also referred as THV) such that the commissures of the prosthetic heart valve are minimally misaligned to the commissures of a native heart valve in a manner which is novel and user friendly.

The potential benefits of CA include (a) improved hemodynamic performance due to balancing of flow dynamics within neo-sinus, (b) reduced leaflet stress and improved long term durability of THV, (c) unrestricted access to coronary ostia for future re-interventions (PCI) and (d) ability to undertake the Basilica procedure for future valve-in-valve interventions. The foregoing features and other features as well as the advantages of the invention will become more apparent from the following detailed description, which proceeds with reference to the accompanying figures.

The system and method to achieve commissural alignment (CA) is based on following basic approach.
1. Determination of the Angle of Commissural Alignment (AoCA). This angle depends solely on the anatomy of the native aortic valve of the patient where the THV is to be implanted. The method of determination of AoCA is described in the detailed description below.
2. Crimping the THV on the delivery system such that one of the commissures of the THV is axially aligned with the AoCA determined as in 1 above with aligner/s facing upward. The method of crimping to achieve this orientation is described in the detailed description below.
3. Implanting the THV crimped in the manner described in 2 above by keeping the aligner/s pointing upward during the entire procedure. The method of implantation is described in the detailed description below.

This basic approach is applicable to a balloon expandable or a self-expanding THV as described in the detailed description below.

### BRIEF DESCRIPTION OF DRAWINGS

The summary above, as well as the following detailed description of illustrative embodiments, is better understood when read in conjunction with the appended figures. For the purpose of illustrating the present disclosure, exemplary constructions of the disclosure are shown in the figures. However, the disclosure is not limited to specific methods and instrumentalities disclosed herein. Moreover, those familiar with the art will understand that the figures are not to scale. Wherever possible, like elements have been indicated by identical numbers.
FIG. 1 is a schematic representation of native aortic root.
Fig. 2 shows a schematic representation of anatomic/AP view of the aortic root schematically. It also shows the location where cross-section A-A of Mid-Sinuses of Valsalva (Mid-SoV) should be taken.
Fig. 3 shows a multi-slice computed tomography (MSCT) cross-section of mid-sinuses of Valsalva in a short axis perspective.
Figs. 3A illustrates an echo image of MSCT image of Fig. 3.
Fig. 4 illustrates the MSCT image of Fig. 3 schematically.
Figs. 5 to 10 illustrate method of determining Angle of Commissural Alignment (AoCA) using MSCT imaging using a software such as "3mensio^{™}".
Figs 11 to 14 (11A-D, 12A-D, 13A-B, 14A-B) illustrate superimposed clock-face with angle markings for various anatomies.
Fig. 15 shows a frame of a typical exemplary balloon expandable prosthetic valve in perspective view.
Fig. 16 depict a typical exemplary delivery system for a balloon expandable THV.
Fig. 17 depicts the proximal end of the delivery system of Fig. 16 and handle located at this end.
Fig. 18 depicts the distal end of the delivery system of Fig. 16 showing the inflatable balloon and tip.
Figs. 19A-D depict a typical exemplary crimper with angle markings on its face for crimping a balloon expandable THV on a balloon of a delivery catheter.
Fig. 20 illustrates the crimper with the balloon expandable prosthetic valve located inside the iris opening of the crimper and the orientation of the balloon catheter as it is being inserted into the iris opening.
Figs. 21A-D, 22A and 22B illustrate a confirmation gauge in various views.
Figs. 23A and 23B illustrate a method of confirming the correctness of positioning of THV on the balloon of a delivery catheter before final crimping.
Fig. 24 depicts ideal alignment of the commissures of a THV after implantation with the commissures of the native aortic valve using the system and method of this invention.
Fig. 25 shows an exemplary illustration of a typical self-expandable prosthetic heart valve.
Figs. 26 shows distal portion of a typical delivery system of Fig. 27 for a self- expandable prosthetic valve.
Fig. 26A shows cross sectional view of the distal portion of the delivery system of Fig. 27.
Fig. 26B shows perspective view of the distal portion of the delivery system of Fig. 27.
Fig. 27 shows assembly of a typical delivery system for a self-expanding THV.
Fig. 28 shows the distal portion of the delivery system of Fig. 26 with continuous aligner marked on the outer shaft.
Figs. 29A and 29B show a confirmation gauge of Figs. 21A-D, and 22A-B with AoCA marked on it. Fig. 29B shows the confirmation gauge of Figs. 21C and 21D with full angle markings.
Figs 30, and 30A depict how the distal end of the delivery catheter is introduced into the central opening of the confirmation gauge and method of orienting the holder.
Fig. 31, 31A/B, 32 and 32A/B illustrate a method for orienting a self-expandable THV when crimped on the inner lumen of the delivery catheter using the confirmation gauge.
The features of the described embodiments are as included in the claims. The embodiments and claims are best understood by referring to the description that follows and the figures accompanying the description.

### DETAILED DESCRIPTION OF ACCOMPANYING DRAWINGS

Prior to describing the invention in detail, definitions of certain words or phrases used throughout this patent document will be defined: the terms "include" and "comprise", as well as derivatives thereof, mean inclusion without limitation; the term "or" is inclusive, meaning and/or; the phrases "coupled with" and "associated therewith", as well as derivatives thereof, may mean to include, be included within, interconnect with, contain, be contained within, connect to or with, couple to or with, be communicable with, cooperate with, interleave, juxtapose, be proximate to, be bound to or with, have a property of, or the like; Definitions of certain words and phrases are provided throughout this patent document, and those of ordinary skill in the art will understand that such definitions apply in many, if not most, instances to prior as well as future uses of such defined words and phrases.

Reference throughout this specification to "one embodiment," "an embodiment," or similar language means that a particular feature, structure, or characteristic described in connection with the embodiment is included in at least one embodiment. Thus, appearances of the phrases "in one embodiment," "in an embodiment," and similar language throughout this specification may, but do not necessarily, all refer to the same embodiment, but mean "one or more but not all embodiments" unless expressly specified otherwise. The terms "including," "comprising," "having," and variations thereof mean "including but not limited to" unless expressly specified otherwise. An enumerated listing of items does not imply that any or all of the items are mutually exclusive and/or mutually inclusive, unless expressly specified otherwise. The terms "a," "an," and "the" also refer to "one or more" unless expressly specified otherwise.

The description includes a number of exemplary embodiments which are provided for illustration of a general category of devices. It is understood that other alternative designs/variants of these exemplary embodiments are possible and are deemed to be included in the description and scope of this invention.

Although the operations of exemplary embodiments of the disclosed method may be described in a particular, sequential order for convenient presentation, it should be understood that the disclosed embodiments can encompass an order of operations other than the particular, sequential order disclosed. For example, operations described sequentially may in some cases be rearranged or performed concurrently. Further, descriptions and disclosures provided in association with one particular embodiment are not limited to that embodiment, and may be applied to any embodiment disclosed herein. Moreover, for the sake of simplicity, the attached figures may not show the various ways in which the disclosed system, method, and apparatus can be used in combination with other systems, methods, and apparatuses.

Furthermore, the described features, advantages, and characteristics of the embodiments may be combined in any suitable manner. One skilled in the relevant art will recognize that the embodiments may be practiced without one or more of the specific features or advantages of a particular embodiment. In other instances, additional features and advantages may be recognized in certain embodiments that may not be present in all embodiments. These features and advantages of the embodiments will become more fully apparent from the following description and apportioned claims, or may be learned by the practice of embodiments as set forth hereinafter.

It should be noted that terms like 'prosthetic aortic valve', 'prosthetic valve', 'prosthetic heart valve' and 'transcatheter heart valve' (THV) correspond to the same implantation device and hence, are interchangeably referred throughout the description.

In the description of the system and methods, "proximal" shall mean the direction towards the operator performing the procedure and "distal" shall mean the direction away from the operator.

The present invention discloses a commissural alignment system for a trans-catheter heart valve when used for replacing a diseased native aortic valve. The system and method described herein can be used for a balloon expandable as well as for a self-expandable prosthetic valve using same basic procedure as under.
- Determine the Angle of Commissural Alignment (AoCA).
- Crimp the prosthetic valve on the delivery catheter with the help of a crimper; using AoCA, the aligners marked on the outer shaft of the catheter, angle markings on the crimper/confirmation gauge and one of the commissures of the prosthetic valve as guiding features.
- Implant the prosthetic valve at the target location taking care that the aligner/s marked on the outer shaft of the delivery catheter points upward during entire implantation procedure.
Though the system and method are described separately for balloon-expandable and self-expanding prosthetic valves for clarity, a skilled person would readily recognize that the basic principles used for both types of prosthetic valves are identical.

The commissural alignment system of the present invention can be used for implanting a balloon expandable prosthetic aortic valve or a self-expandable prosthetic aortic valve (a transcatheter heart valve, THV) in a patient's body to achieve minimum misalignment of commissures of the prosthetic aortic valve with the commissures of the native aortic valve. The present invention achieves the commissural alignment by introducing novel modifications in a delivery system and a crimper that are easy to incorporate. A skilled person would immediately appreciate that these modifications do not affect the basic design and constructional features of the delivery system and the crimping system. As would be evident, the modifications are very similar for balloon expandable as well as self expanding THVs.

Further, the present invention discloses a crimping method based on the above modifications that is easy for a user to follow and also assists in nearly perfect alignment of commissures of a prosthetic heart valve with the native aortic valve.

The description below is divided into following three sections for convenience and better understanding of the system and the method.
- Determination of AoCA based on patient's anatomy of aortic root.
- System and method for balloon expandable prosthetic valve.
- System and method for self-expanding prosthetic valve.

### Determination of AoCA

In a native tricuspid aortic valve anatomy, the three commissures are located at 120° with respect to each other. For True Type-0 bicuspid anatomy, these commissures are located at 180° with respect to each other. Ordinarily, there are three coronary cusps at the aortic root. Ideally, the coronary arteries originate from coronary cusps.

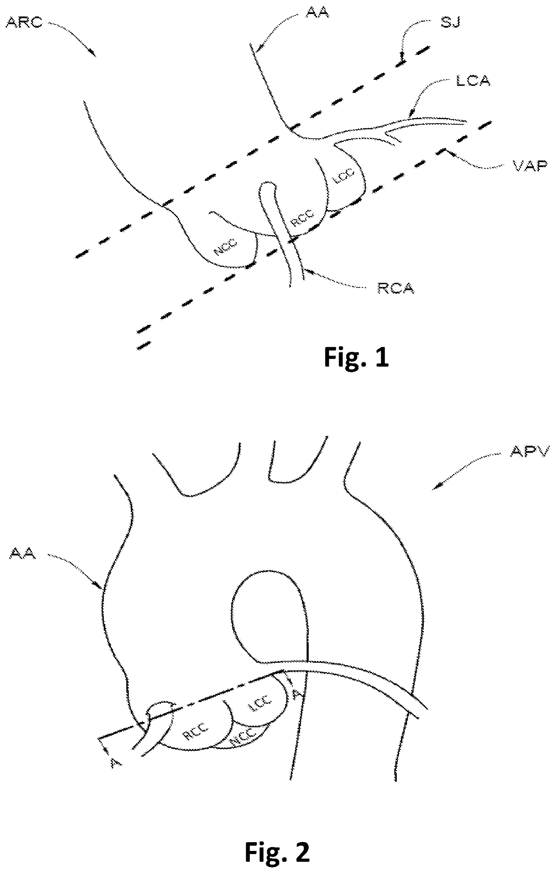

Aortic root complex (ARC) is schematically shown in Fig. 1. Aortic root complex is the first part of aorta attached to the heart towards the exit of left ventricular outflow tract (LVOT). It is a part of the ascending aorta (AA) containing the native aortic valve and other anatomical structures such as - Sinuses of Valsalva (SoV), Sino-tubular junction (SJ), coronary arteries. Normally, there are three cusps of the native aortic valve. The coronary arteries originate from the vicinity of the aortic sinus bulb from two of the three cusps. The Right Coronary Artery (RCA) ideally originates from Right Coronary Cusp (RCC) and the Left Coronary Artery (LCA) originates ideally from Left Coronary Cusp (LCC). The remaining cusp is termed Non-Coronary Cusp (NCC) as no coronary artery originates in the vicinity of this cusp. Typically, all the 3 coronary cusps are in different planes with NCC residing lower than either RCC or LCC. In a 2-dimensional view, there are two distinct demarcation cross-sectional planes. The first plane, at the origin of aortic root, is termed the Virtual Annular Plane (VAP) and the second plane, at the origin of ascending aortic, is termed the Sino tubular Junction (SJ). In a standard TAVR/TAVI procedure staged under fluoroscopic guidance, the native coronary cusps are made co-planar wherein the hinge point of each cusp is in a straight line and all the three cusps are well separated without any parallax. The VAP thus achieved can be visible under fluoroscopy (normally by placing a standard 5Fr pigtail catheter in the NCC during aortogram) and is a guiding feature to position the prosthetic valve at the optimal location for implantation. Fig. 1 depicts the aortic root with all the 3 cusps aligned along the virtual annular plane (VAP), along with Sinotubular junction (SJ) and coronary arteries originating from cusps.

Fig. 2 shows anatomic/AP view (APV) of the aortic root of Fig. 1 schematically. It also shows location where cross-section A-A of Mid-Sinuses of Valsalva (mid-SoV) should be taken for determining AoCA.

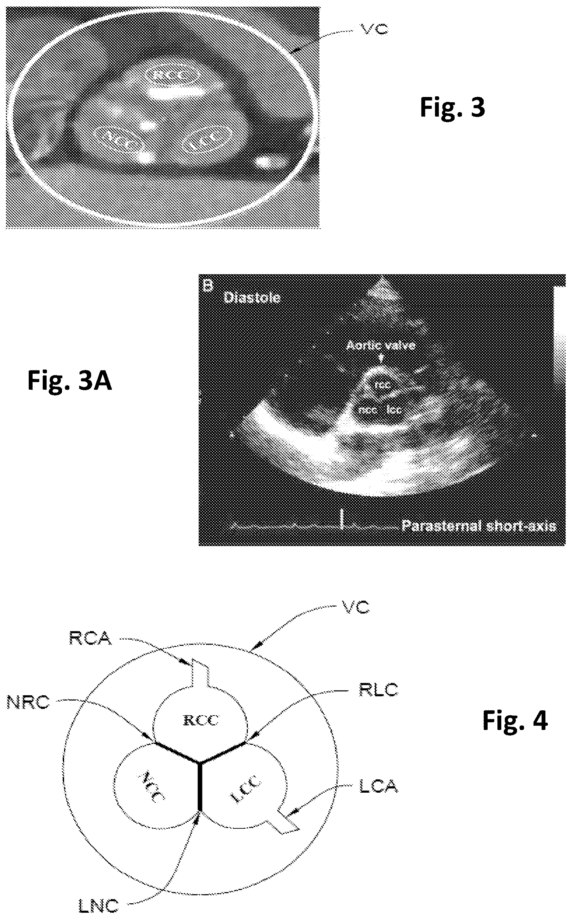

Fig. 3 shows fluoroscopy image using Multi-slice computed tomography (MSCT) at cross-section A-A of Mid-Sinuses of Valsalva (refer to Fig. 2) in a short-axis perspective. Fig. 3A shows Echo image of what is seen in Fig. 3. A virtual circle (VC) is drawn by the software around the fluoroscopy image as shown in Fig. 3.

Fig. 4 shows image of Fig. 3 schematically to get clarity. Fig. 4 shows three cusps viz. RCC, LCC and NCC with coronary arteries RCA and LCA originating from RCC and LCC shown as short projections. No artery originates from NCC. There are three commissures of the native aortic valve designated as RL-commissure (RLC), LN-commissure (LNC) and NR-commissure (NRC). The virtual circle (VC) drawn by the software is also shown.

It may be noted that the fluoroscopic view, as observed in Multi-slice Computed Tomography (MSCT) or any other equivalent imaging system, is a mirror image of the true anatomical/AP view. This fact is known to the surgeon performing the procedure.

This invention discloses a novel method to achieve positioning of the prosthetic valve such that the commissures of the prosthetic valve are minimally misaligned to those of the native aortic valve. The method can be easily followed by the user. The alignment is achieved by aligning any one of the three commissures of the THV preferably with the mid-sinus of RCC as seen in a cross-sectional image derived from MSCT or an equivalent imaging system. Alternatively, the alignment may also be achieved by aligning any one of the three commissures of the THV with mid-sinus of LCC or NCC. The method of this invention can be used for a balloon expandable as well as a self-expanding THV.

This invention also discloses novel additional features that may be easily incorporated in the delivery and crimping systems as well as the method of implantation which is easy to follow to achieve positioning of the prosthetic valve such that the commissures of the prosthetic valve are minimally misaligned to those of the native aortic valve.

### A method of determining AoCA:

To begin with, "Angle of Commissure Alignment" (AoCA) is determined by examining anatomy of a patient's aortic root which houses native aortic valve. This is done by using technique outlined as step wise procedure described further below. The following procedure is described with respect to mid-sinus of RCC as reference point. However, mid-sinus of LCC or NCC may also be used as reference point. The AoCA plays very important role in achieving commissural alignment.

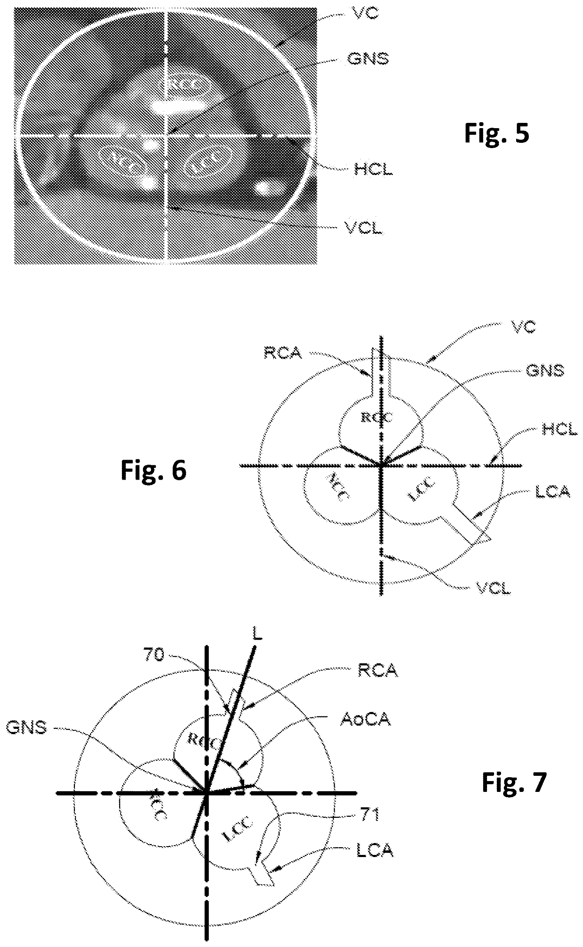

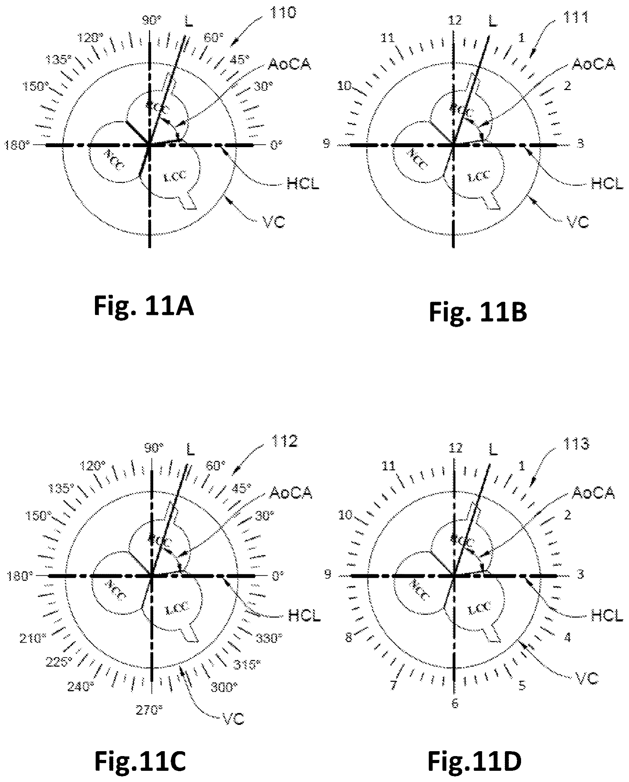

For explaining the technique to determine AoCA, MSCT images are used. However, any other similar imaging system can be used in place of MSCT. For clarity, schematic drawings of SoV are also shown in addition to or in place of MSCT cross-section.
- Step-1: Capture the transverse cross-sectional image of the cross-section of mid-Sinuses of Valsalve (SoV) in the MSCT imaging software such as "3mensio^{™}" or any other equivalent software. Fig. 3 shows fluoroscopy image captured by the aforesaid software, along with an echo image in Fig. 3A. The fluoroscopy image of Fig. 3 is shown schematically in Fig. 4 for clarity. As shown in Figs. 3 and 4, a virtual circle (VC) is drawn by the software around the MSCT cross-sectional image of SoV.
- Step-2: Draw horizontal and vertical center-lines (HCL and VCL respectively) on the virtual circle (VC) drawn by the software in the image of Fig. 3 as shown in Fig. 5 and schematically in Fig. 6. The intersection of these center-lines is termed "geometric nodule of sinuses" (GNS). In addition, Fig. 6 shows RCA and LCA originating from respective cusps as short projections.
- Step-3: Draw a line (L) through the geometric nodule of the sinuses (GNS) to the geometric mid-point of RCC (70) as shown in schematic Fig. 7. The angle formed between this line and the horizontal center-line (HCL) extending on right hand side of GNS is termed as "Angle of Commissural alignment (AoCA)". Geometric mid-point of LCC is shown as 71.
- Step-4: Superimpose a face (110) with angles marked on it over the cross-sectional image, e.g. of Fig.7 as shown in schematic drawing Fig. 11A where the angles on the angle markings are expressed as degrees. In this exemplary figure, AoCA is around 71 degrees. In another exemplary embodiment of Fig. 11B, the angle markings are expressed in a more convenient way in the form of a clock (111) over the cross-sectional image e.g. of Fig. 7. In this case, the AoCA is identified as a "Clock-Angle" which may be expressed as, for example, 3:03 O'clock in Fig. 11B.
- The angle markings shown in Figs. 11A and 11B cover half of the circumference of the virtual circle (VC) drawn by the software. If required, the angle markings (112 and 113) may cover the entire circumference of the virtual circle (VC) drawn by the software as shown in Figs. 11C and 11D. Fig. 11C shows angle markings expressed in degrees, while Fig. 11D shows angle markings expressed as clock angle.

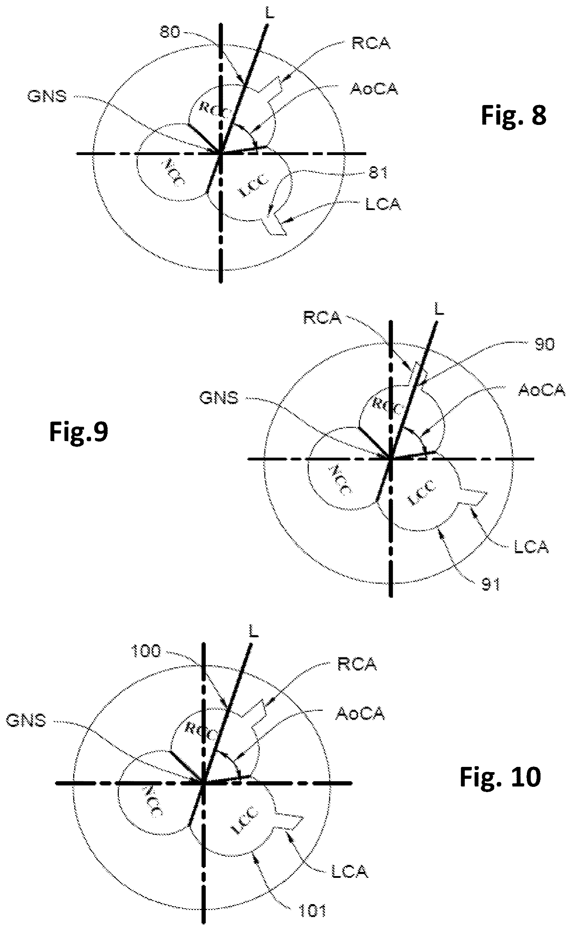

Fig. 7 shows one of the cases where the Right Coronary Artery (RCA) originates from the geometric mid-point of RCC (70) and the Left Coronary Artery (LCA) also originates from the geometric mid-pont of LCC (71). In other cases, RCA may originate off-centered as shown schematically in Fig. 8 where the geometric mid-point of RCC is shown as 80. In this case also, AoCA is still the angle formed between a line drawn from GNS through the geometric mid-point of RCC (80) and the horizontal center-line extending on right hand side of the GNS as shown in Fig. 8. Other exemplary anatomies are shown in Figs. 9 and 10. In Fig. 9, the LCA originates off-centered from the geometric mid point (90) of LCC) and in Fig. 10, both RCA and LCA originate off-centered from geometric mid points of RCC (100) and LCC (101) respectively. There would be other possible anatomical variations. In all these cases, AoCA is still the angle formed between a line (L) drawn from GNS through the geometric mid-point of RCC and the horizontal center-line extending from GNS on right hand side, irrespective of where the coronary arteries are generated from. Hence, this procedure would work effectively in case of such differences in anatomy.

In all the embodiments described above, AoCA is measured between the line L and the horizontal center line (HCL) extending on right hand side of the GNS. However, AoCA may also be measured between line L and the horizontal center line extending on left hand side of the GNS. Similarly, AoCA may also be measured between line L and the vertical center line extending upwards or downwards from the GNS. The main point is to measure AoCA. In such cases, the reference point will change. In the following description, AoCA is measured between line L and the horizontal center line on the right-hand side of GNS. A skilled person will readily understand how the method can be used with other reference points.

Two alternate units of measuring and expressing AoCA are described above viz. angle in degrees and as clock angle. A skilled person would appreciate that any other unit of measuring and expressing this angle (i.e. AoCA) is equally effective and can be used. The Clock-Angle is a convenient way to measure and express the angle. The significance of AoCA would be clear from the description of the crimper which follows.

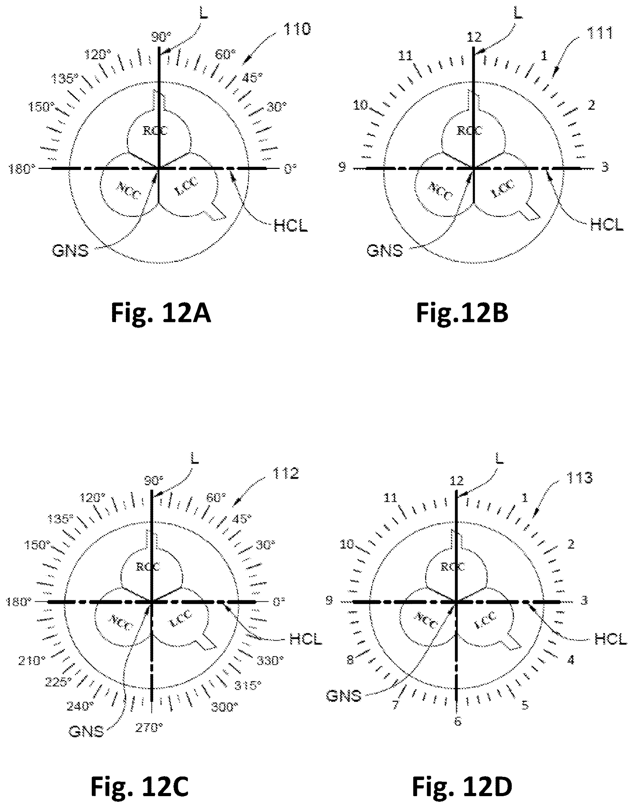

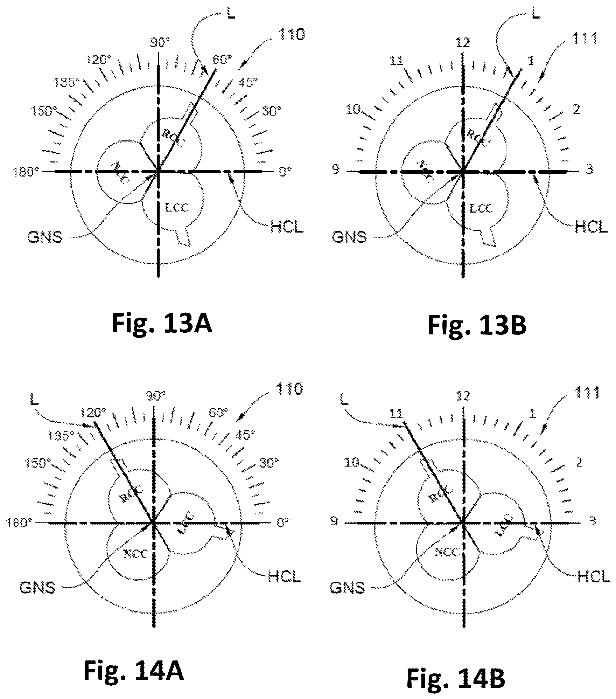

Anatomy is patient-specific and hence each patient would have different anatomy. Hence it is necessary to determine AoCA of the patient being treated. Figures 12A, 13A, and 14A show examples of a few anatomical variants of Mid-RCC corresponding to the angles in degrees. Figures 12B, 13B and 14B show examples of the anatomical variants shown in Figs. 12A, 13A and 14A respectively corresponding to Clock-Angles. In these exemplary images, AoCA is measured between line L and the horizontal center line (HCL) extending on right hand side of the GNS. These images are illustrative and show one of the cases where the Right Coronary Artery (RCA) originates from the geometric mid-point of RCC and the Left Coronary Artery (LCA) originates from the geometric mid-point of LCC. In other cases, as described above, RCA and LCA may originate off-centered as mentioned above. In addition, any other unit of measuring and expressing the angle is equally effective. AoCA in case of Fig. 12A, for example, can be expressed as 90°. While in exemplary cases of Fig. 13A and Fig. 14A, it can be expressed as 60° and 120° respectively. AoCA in case of Fig. 12B, for example, can be expressed as 3:00 O'clock. While in exemplary cases of Fig. 13B and Fig. 14B, it can be expressed as 3:05 and 2:55 O'clock respectively.

Figs. 12C and 12D show the angle markings covering the entire VC in degrees and clock angles respectively for illustration.

The above description relates to a specific situation wherein the AoCA is determined with respect to geometric midpoint of RCC. A skilled person would understand that AoCA may also be determined from geometric midpoint of LCC or NCC also. It may be noted that AoCA in these cases would be different than that measured with respect to geometric midpoint of RCC. The reference will then shift to LCC or NCC throughout.

The skilled person would readily understand that this method is also applicable for different types of bicuspid aortic valve anatomy. The AoCA will be determined in similar manner.

As would be clear to a skilled person, method of determination of AoCA does not depend on whether the THV to be implanted is balloon-expandable or self-expandable because AoCA is dependent entirely on the anatomy of the patient being treated. Hence, the method of determining AoCA is applicable to both the types of THVs. In addition, the aim is to measure AoCA with any specific reference point as mentioned above.

### Method for commissure alignment for a balloon expandable THV

### Balloon expandable THV

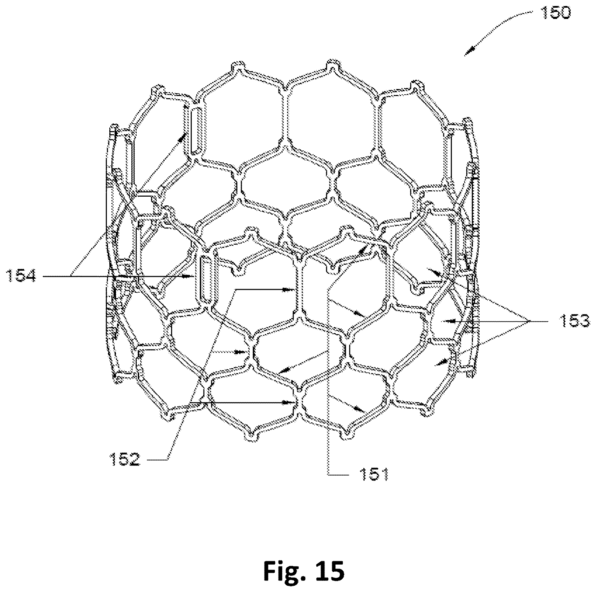

A person skilled in art is well aware of different designs of a balloon expandable prosthetic valve which consists of, amongst other components, a frame which is radially collapsible and expandable. The frame is a scaffold structure preferably of tubular shape, formed generally by multiple rows of circumferentially extending struts which may be interconnected either to each other directly or by struts extending in general axial direction. The scaffold structure formed by the struts forms multiple rows of cells. There are a number of balloon expandable prosthetic valves available in the market and described in literature with different scaffold designs. The designs continue to be refined and optimized. A skilled person will readily understand that this invention can be used for the frame of any scaffold design. For illustration, a typical exemplary frame of a balloon expandable THV is shown in Fig. 15. As shown in Fig. 15, the exemplary frame 150 of a balloon expandable prosthetic valve has circumferentially extending rows of struts 151 joined by generally vertically oriented struts 152. These struts form rows of cells 153. In addition, there are commissure attachment areas 154 where commissure portions of the leaflets are attached. It is understood that Fig. 15 shows merely an illustration of a frame 150 of a balloon expandable THV.

The balloon expandable THV is further provided with at least two leaflets, preferably three leaflets made from animal tissue or synthetic materials. The commissure portions of two adjacent leaflets are attached to the commissure attachment areas 154 of the frame 150 to form commissures of the prosthetic valve. The frame 150 can be made from metallic or polymeric materials.

The balloon expandable prosthetic aortic valve may further include at least one of an internal skirt and an external skirt. The internal skirt covers the internal surface of the frame 150 at least partially. The external skirt covers the external surface of the frame 150 at least partially.

A skilled person will readily understand that this invention can be used for a balloon expandable prosthetic valve of any design.

### Delivery system for balloon expandable THV:

As mentioned previously, to achieve Commissural Alignment, novel additional features are required to be provided on the delivery system. This section describes these additional features for a delivery system for a balloon-expandable THV i.e. a balloon catheter.

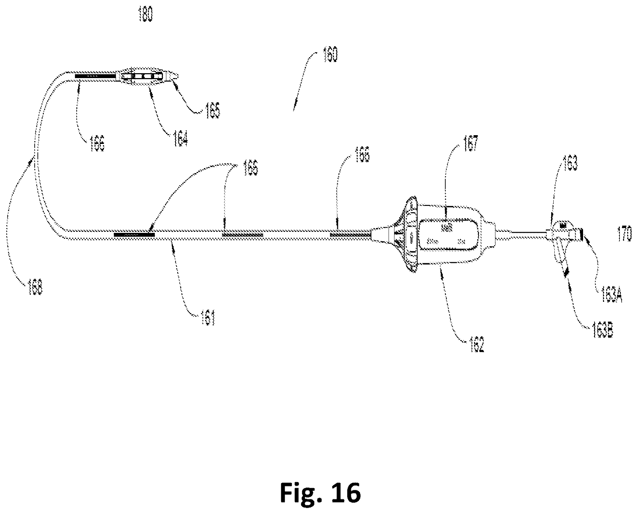

A skilled person is well aware of the construction of a balloon catheter used for radially expanding a balloon expandable device such as a stent or a prosthetic valve. An exemplary balloon catheter 160 is shown in Fig. 16 has a proximal end 170 and a distal end 180, an elongated external tube 161 (also referred to as "outer shaft") through which extends an internal tube (also referred to as "inner lumen", not shown) coaxially with the external tube. Both these tubes are collectively referred to as "tubes". The tubes have respective distal end and proximal end.

The proximal ends of the tubes pass through a handle 162 and are attached to a Y-connector 163 that has an exit port for guidewire 163A and a port for injecting inflation fluid 163B. Guidewire port is in communication with the inner lumen and the port for inflation fluid is in communication with the annular space between the two tubes.

The balloon 164 is attached to the distal end of the outer shaft 161. The inner lumen extends through the balloon 164 and it ends into a soft tip 165 at the distal-most end of the catheter. Guidewire enters the guidewire lumen at the distal soft tip 165 of the catheter, enters the inner lumen which passes through the balloon 164 and exits at the Y-connector 163. As stated previously, 'proximal' means towards the operator, while 'distal' means away from the operator.

The balloon 164 is radially expanded by injecting pressurized inflation fluid into the balloon 164 through the annular space between the outer shaft 161 and the inner lumen.

The above description discloses details of a balloon catheter 160 in accordance with an exemplary embodiment. It should be noted that there may be other designs of balloon catheter which are deemed to be covered under this invention.

The present invention involves novel changes that can be easily made in a balloon catheter, as described below, and help in commissural alignment.

The outer shaft 161 of the delivery system is provided with one or more than one marking/s termed as "aligner/s" 166. The embodiment of Fig. 16 shows multiple aligners 166, spaced apart from each other, preferably equidistant from each other in a single orientation i.e. on the same axis. The subsequent discussion refers to plurality of aligners for convenience. However, it is understood that it includes a single aligner as well. As the aligners 166 are provided on the outer shaft 161 of the delivery system 160, they are visible as such. To improve plain visibility, the colour of the aligners 166 should contrast the colour of the outer shaft 161. The aligners would be visible also under fluoroscopy if they are made from radiopaque material. The aligners may either be painted on the outer shaft 161 or strips of biocompatible material may be pasted on the outer shaft 161. If the paint is radiopaque or the strips are made from a radiopaque material, the aligners will be visible under fluoroscopy as well. Alternately, the aligners may be provided on the outer shaft 161 by using a laser beam. Any other method of providing the aligners is equally effective. The aligners should be biocompatible.

A preferred embodiment of delivery system of this invention that has 120 cm working length is provided with four aligners 166, each of equal length and are spaced uniformly apart from each other. Alternately the number of aligners may be more than or less than four and spacing between them may be non-uniform. Alternately, there may be a single aligner provided as a continuous line extending from the proximal handle to the distal end of the outer shaft. The colour of the outer shaft 161, for example, of the preferred embodiment is orange. Hence, the colour of the aligners 166 may be white. The color of the outer shaft of the delivery system being in contrast with the colour of the aligner/s provides improved visibility to the aligners. The aligners of the preferred embodiment are made of white biocompatible strips which may preferably be radiopaque. The strips may be fixed onto the outer shaft 161 by known techniques such as pasting. Alternately, the white aligners may be painted on the orange outer shaft 161.

As the aligners 166 are provided on a single axis, they help in maintaining specific orientation during introduction of the delivery system for commissural alignment. The aligners 166 assist in preventing any inadvertent torqueing of the delivery system during insertion and further tracking the system across the aortic anatomy.

For example, in the preferred embodiment, there are multiple aligners 166 marked on the same axis as that of the company logo 167 (or any other equivalent reference) on the proximal handle as shown in Fig. 16. This is a convenient way of identifying the axis of the aligners 166. Alternately, as mentioned previously, there may be a single aligner throughout its entire length.

Fig. 16 shows a delivery system where a distal section of the shaft 168 can be flexed to follow the shape of aortic arch. It may be noted that this is only an example and the delivery system may have a shaft without flexing option.

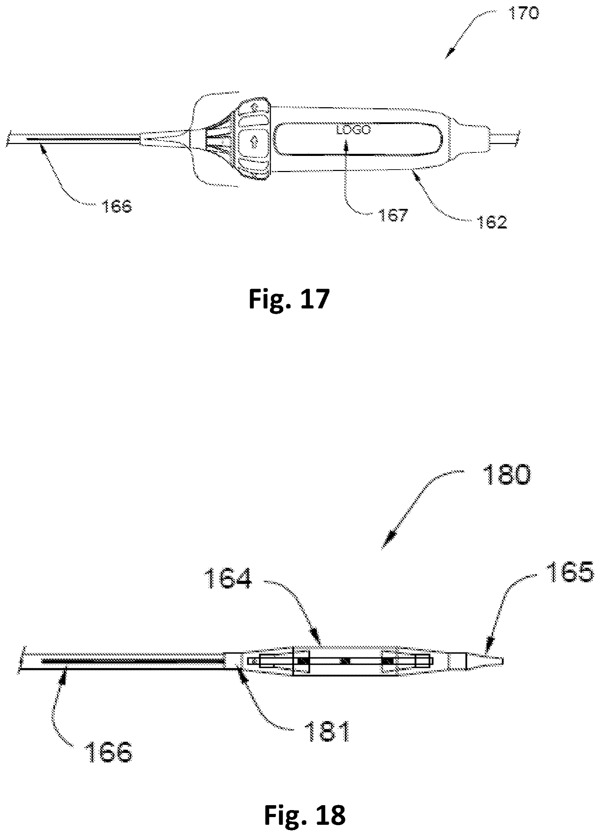

The aligners 166 start at the proximal handle 162 (also referred as proximal most aligner) with reference to an axis and end at the distal end of the outer shaft 161 (namely, distal most aligner) or the proximal edge of the balloon 164 maintaining the same axis. Fig. 17 shows preferred embodiment of proximal end 170 of balloon catheter 160. In this preferred embodiment, the aligners 166 follow the same axis as the company logo 167 on the proximal handle 162 and continue on the same axis (i.e. retain same orientation) till proximal edge of the balloon 164 or distal end 180 of the outer shaft 161 as shown in Fig. 18 which shows distal end 180 of the balloon catheter 160. This is merely a convenient way of marking the aligners 166. It

is not necessary to follow the same axis as company logo. Alternatively, there may be a single aligner starting at the proximal handle with reference to an axis and ending at the proximal edge of the balloon 164.

A skilled person would recognize that marking of aligners 166 on the outer shaft as stated above is novel and does not affect the design parameters or performance of the delivery catheter.

### Crimper:

As mentioned previously, to achieve Commissural Alignment, novel additional features are required to be provided on the crimper. This section describes these additional features for a crimper.

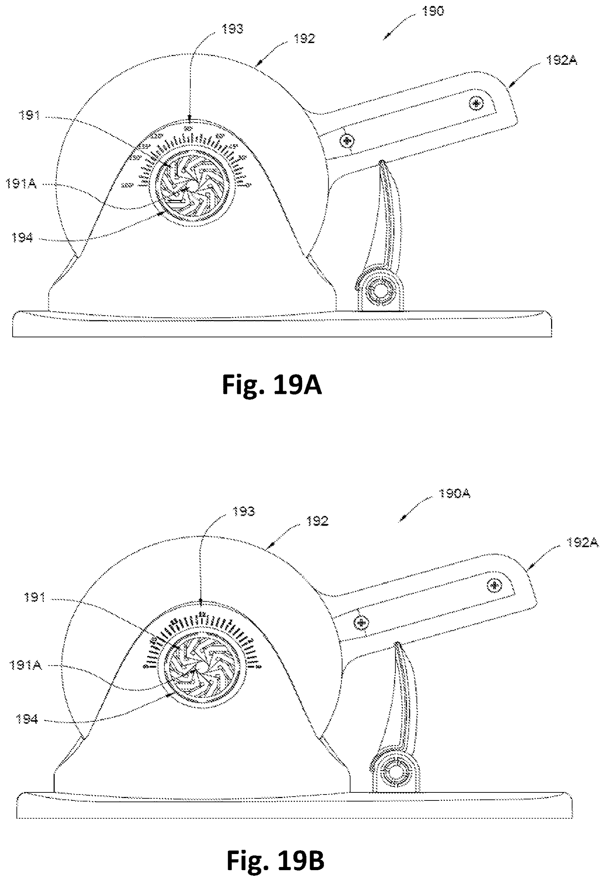

A typical crimper 190 for a balloon expandable prosthesis such as a THV, is shown in Fig. 19A. The exemplary crimper 190 has multiple jaws 191 which are arranged inside a housing 192 such that they form a nearly circular iris opening 191A. Typical crimpers consist of minimum six jaws; normally twelve jaws. However, the crimper may have any number of jaws, six or more than six. The housing 192 houses a mechanism to move these jaws in a synchronous manner by which the diameter of the iris opening 191A formed by the jaws can be varied or adjusted and remains generally circular (i.e. a regular polygon) in shape. The mechanism is operated by a handle 192A. Moving the handle 192A activates the mechanism. Initially, the handle is in a position (normally up) where the diameter of the iris opening 191A is maximum. The prosthetic valve, which is at least in a partially expanded configuration, is positioned over the deflated balloon of the delivery catheter and is introduced into the open iris opening 191A which is large enough to accommodate balloon and the prosthetic valve. The handle is moved (normally downward) to activate the mechanism which moves the jaws 191 such that the diameter of the iris opening 191A formed by them reduces gradually. The reduction in the diameter of the opening causes the diameter of the frame of the prosthetic valve to radially collapse to achieve crimping of the valve on the balloon.

The crimper describe above is exemplary. The procedure described herein is applicable to other types of crimpers operating on similar working principle.

A skilled person is well aware of the crimping procedure and the crimpers of different designs. Conventionally, no attention is paid to the orientation of the commissures of the prosthetic valve while crimping the prosthetic valve on the balloon. Hence, there is no conscious effort to achieve commissural alignment. Novel changes as described below can be easily made on a conventional crimper to achieve commissural alignment. A skilled person would readily understand that such changes do not affect the basic design or the operation of the crimper.

In the present invention, a conventional crimper may be provided with angle markings 193 on at least one of its external surfaces around a central opening 194 of the crimper as shown in Fig. 19A where these markings 193 are expressed in degrees. These angle markings 193 should correspond to the angle markings on the transverse cross-sectional image of mid-SoV in Fig. 11A, 12A, 13A or 14A.

Alternately, the angle markings 193 may be expressed as Clock-Angles as shown in the embodiment 190A of Fig. 19B, corresponding to the angle markings 193 shown in Fig. 11B, 12B, 13B or 14B.

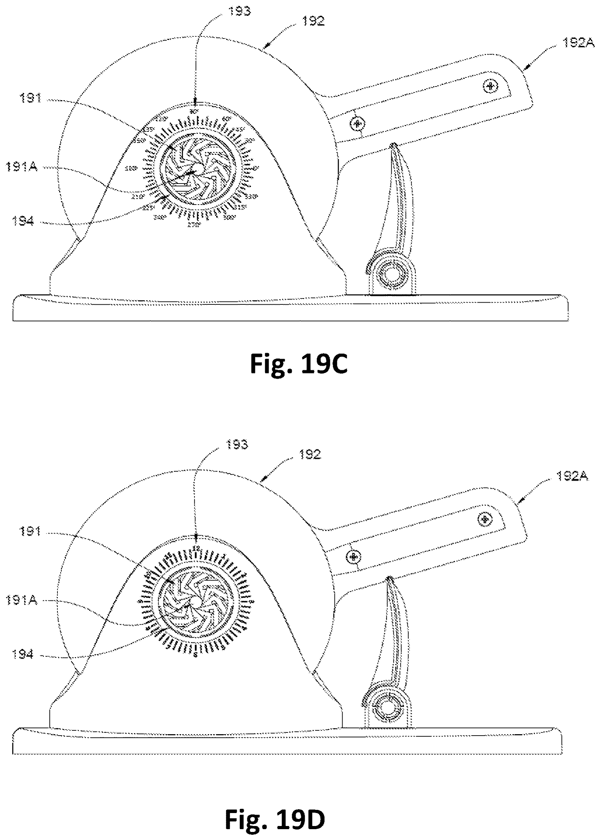

In the embodiments described above, the angle markings 193 cover half of the circumference of the central opening 194 of the crimper. The angle markings 193 may be provided such that they cover the entire circumference of the central opening 194 of the crimper as shown in Figs. 19C and 19D. The angle markings 193 of Fig. 19C are expressed in degrees while they are expressed as clock angles in Fig. 19D. It is understood that these angle markings correspond to the angle markings on the transverse cross-sectional image of mid-SoV (Figs. 11A-D, 12A-D, 13A/B, 14A/B).

As mentioned previously, any other way of measuring and expressing angles is equally effective and the same may be followed for marking the outer surface of a crimper. However, the angle markings 193 on the crimper should correspond to the angle markings on the transverse cross-sectional image of mid-SoV.

The angle markings may be provided on both the sides of the crimper for convenience of either left or right handed person.

### Procedure for achieving Commissural Alignment

Using the AoCA determined by the above method, aligners 166 on the delivery system described above, and angle markings 193 provided on the crimper, a surgeon can achieve commissural alignment in a manner which is easy to follow as described in the procedure below. This procedure mentions Clock-Angle as the basis for achieving commissure alignment as it is very convenient to use. However, a skilled person will understand that measuring and expressing AoCA in degrees or any other way is equally effective and can be used for the procedure and fall under the scope of this invention. It is understood that the following procedure is performed under aseptic condition.

The procedure described is for a tri-leaflet valve with three commissures positioned at 120° to each other. However, this procedure is applicable to a bi-leaflet valve with two commissures or any other anatomical variation as per Sievers classification of bicuspid aortic valves.

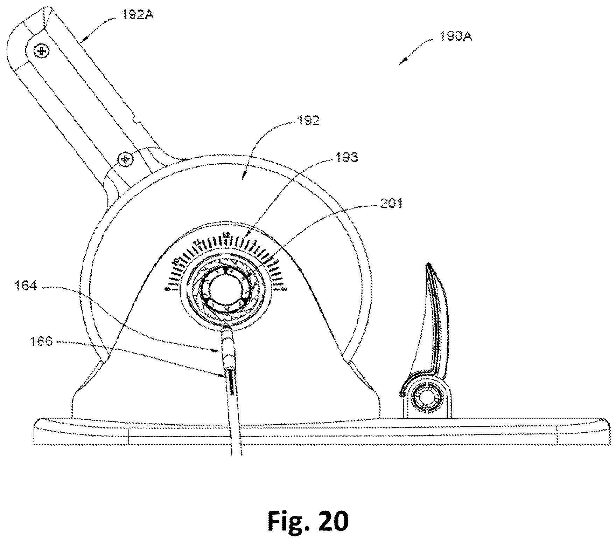

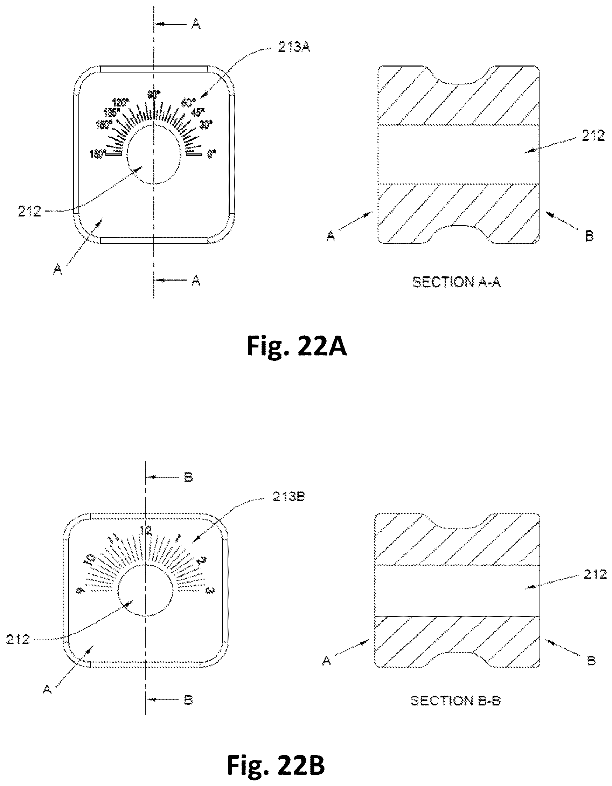

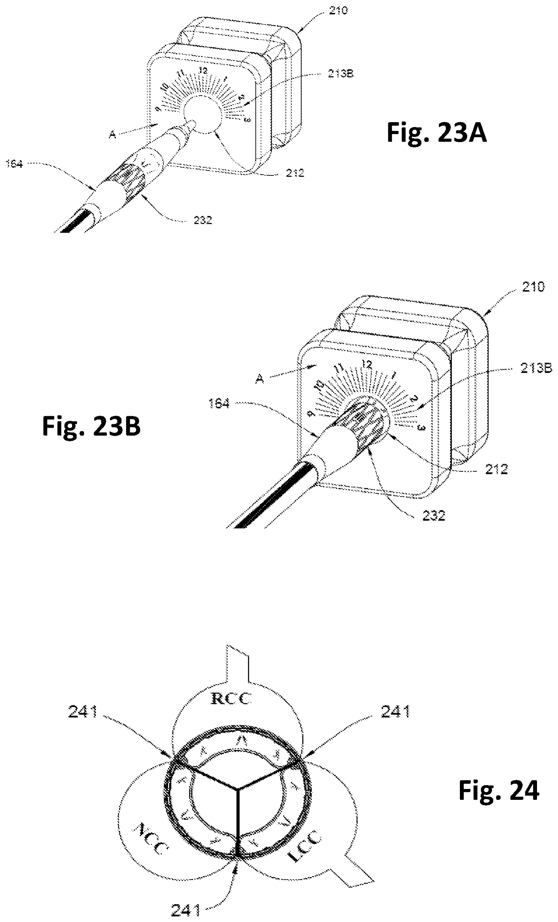

The step wise procedure is as under.
1. Determine AoCA of the patient by the procedure described previously and compute corresponding Clock-Angle.
2. Place the crimper with angle markings 193 expressed as clock-angles on the preparation table and open the iris opening 191A by turning the handle of the crimper.
3. As shown in Fig. 20, place ready-to-crimp prosthetic valve 201 at least partially across the iris opening 191A of the crimper such that any one of the commissures of the prosthetic valve is aligned to the AoCA(determined in step-1). To hold the prosthetic valve 201 in this position, the diameter of the iris opening 191A may be reduced by operating the handle 192A till the iris opening 191A presses a little on the outer surface of the prosthetic valve 201. This will hold the prosthetic valve in this position till it is crimped.
4. Place the balloon 164 of the delivery system in deflated condition at least partially across the prosthetic valve (which is held inside the iris opening of the crimper) such that the distal edge aligner 166 faces upwards as shown in Fig. 20.
   The aligners 166 should always face upwards irrespective of AoCA. In case there are no aligners 166, the system may be held with proximal handle 162 and company logo 167 facing upwards.
   A gadget may optionally be provided to hold the catheter shaft in the position till the crimping operation is completed to ensure that the aligner always face upward. A skilled person is capable of designing such a gadget.
5. Crimp the prosthetic valve on the balloon of the delivery system maintaining the orientation of one of the commissures of the prosthetic valve aligned with respect to
   Clock-Angle corresponding to AoCA and upward orientation of the aligner on the catheter outer shaft at the same time as detailed above.
6. Prior to complete crimping of the THV, it may be advisable to ascertain the orientations of the prosthetic valve and the catheter shaft. At this stage, the prosthetic valve should be crimped to a stage where it is free enough to rotate around the balloon of the catheter to make minor adjustment if required. A Confirmation Gauge may be used for this purpose.

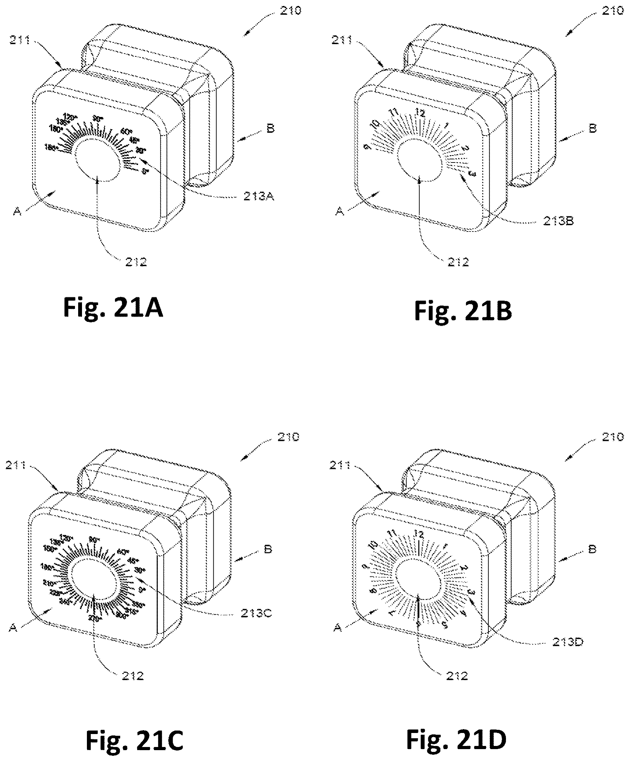

   Figs. 21A-D show four alternate configurations of an exemplary Confirmation Gauge 210. A preferred embodiment of a Confirmation gauge 210 is a square shaped block 211 with a central opening 212 large enough to insert the partially crimped valve along with the balloon. As shown in Fig. 21A, angle markings 213A are provided around the central opening 212. These angle markings 213A correspond to the angle markings 110 on the software images (e.g. 11A, 12A, 13A, 14A) as well as angle markings 193 provided on the crimper (e.g. Fig. 19A). Angle markings 213A are made on at least one of the sides (e.g. side A) of the block from where the crimped THV is inserted into the central opening. Optionally, the angle markings 213A may be provided on both the sides (side A as well as side B) of the exemplary confirmation gauge 210 taking care that they exactly correspond to each other. The preferred embodiment of the exemplary Confirmation Gauge 210 depicted in the Fig. 21A has angle markings 213A in degrees. The embodiment of Fig. 21B has angle markings 213B in clock angles. In both these embodiments, the angle markings 213A and 213B cover half of the circumference of the central opening 212. However, as mentioned previously, any other method of angle markings is equally effective.
   The alternate embodiments shown in Figs. 21C and 21D, the angle markings 213C and 213D respectively, cover the entire circumference of the central opening 212. The angle markings are expressed in degrees (213C) in Fig. 21C and as clock angles (213D) in Fig. 21D. In any case, the angle markings on the confirmation gauge should correspond to the angle markings 193 on the crimper.
   A side view and sectional view of the exemplary confirmation gauge 210 of Fig. 21A and Fig. 21B are shown in Fig. 22A and 22B respectively. The sectional views and side views of embodiments of Figs. 21C and 21D would be similar with angle markings covering the entire circumference of the central opening 212.
   The confirmation gauge may be made from any suitable material such as metal or polymeric material. The shape of the confirmation gauge of the preferred embodiment is square. However, the shape may be rectangular or circular or any other shape.
   The partially crimped THV (232) along with the balloon (164) of the catheter is removed from the iris opening 191A of the crimper and inserted into the central opening 212 of the confirmation gauge 210 with the distal edge aligner facing upwards as shown in Figs. 23A and 23B. For illustration, the angle markings 213B on the face A of the confirmation gauge are expressed as clock angles covering half of the circumference of the central opening 212. The location of any one of the commissure areas of the THV 232 should align with the angle marking 213B corresponding to AoCA (clock-angle in this exemplary embodiment). If not, adjustment may be made in the orientation of the THV 232 to correct the orientation such that one of the commissure areas of the THV 232 aligns with the angle marking 213B corresponding to AoCA. The balloon 164 along with the partially crimped THV 232 is then removed from the Confirmation gauge. THV 232 is then fully and firmly crimped on the balloon 164 using the crimper.
7. After complete crimping of the THV, open the iris opening 191A of the crimper by operating the handle 192A. Remove the balloon 164 with the prosthetic valve crimped on it from the iris opening of the crimper. Now the system is ready for introducing the catheter into the patient's vasculature (normally through the femoral artery) through its recommended introducer sheath. Other routes as mentioned previously can also be used.
8. Insert the distal end of the catheter with the prosthetic valve crimped on the balloon into the patient's vasculature through an introducer sheath keeping the aligner/s 166 facing upwards. In case there are no aligners, the system may be held with proximal handle and company logo facing upwards. This orientation of the aligner/s 166 should not be changed during the implantation procedure. Hence, the operator is required not to torque the delivery system. The aligner/s 166 actually help the operator un-torque the delivery system in case there is an inadvertent torquing due to anatomical challenges. However, the system may be flexed if it has the flexing mechanism.
9. Once the prosthetic valve and the balloon have crossed the aortic annulus, one of the commissures of the prosthetic valve is expected to align towards a native commissure. The other commissures of the prosthetic valve would automatically align towards other native commissures. The prosthetic valve is then deployed using standard techniques. The expected final deployment with commissural alignment 241 is as shown in Fig. 24 under ideal conditions. Fig. 24 shows one of the cases where the commissures of the prosthetic valve are aligned with native NCC-LCC commissures using mid-RCC technique. Practically, there would be minimal misalignment.

The skilled person would readily understand that this method is also applicable for different types of bicuspid aortic valve anatomy.

### Method for commissure alignment for a self-expanding THV

A skilled person is aware that a self-expanding prosthetic valve is different than a balloon expandable prosthetic valve. The delivery system for a self-expanding prosthetic valve is different than that for a balloon catheter. Hence, the procedure for crimping a self-expanding prosthetic valve over a delivery catheter is different than that for a balloon expandable prosthetic valve. However, the basic principles of achieving commissure alignment for a self-expanding prosthetic valve remains similar to that for a balloon expandable prosthetic valve described previously. Novel additional features that can be easily incorporated are provided on the delivery system and crimping method to achieve the goal of commissural alignment for self-expanding prosthetic valve.

### Self-expanding prosthetic valve:

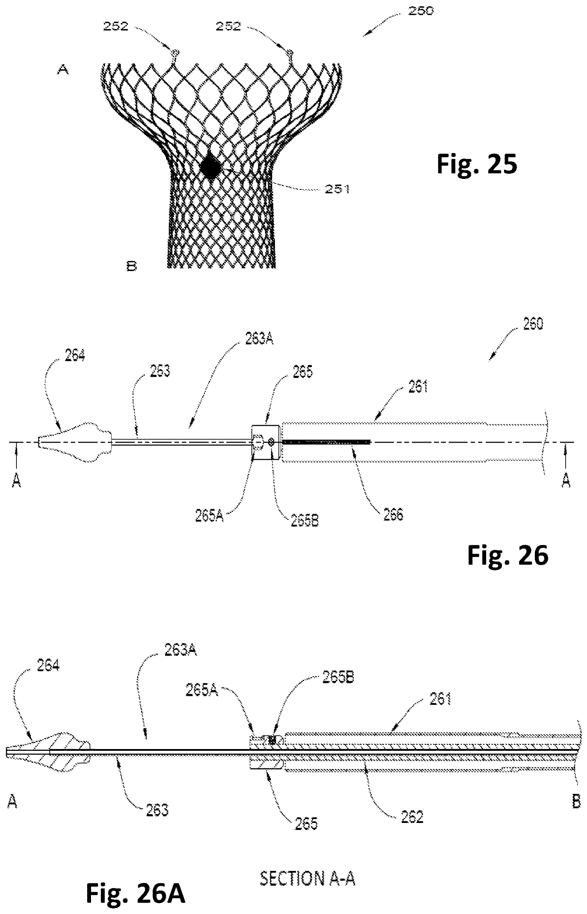

A self-expanding prosthetic valve consists of a frame, generally of tubular shape (but may be with different diameters along its axial length), made from an alloy with shape memory such as nickel-titanium alloy e.g. nitinol or a polymer with shape memory properties. There are a number of self-expanding prosthetic valves available in market or described in the literature with different scaffold designs. The designs continue to be refined and optimized. A skilled person will readily understand that this invention can be used for the frame of any scaffold design. A typical exemplary frame 250 of a self-expanding prosthetic valve is shown in Fig. 25. The frame has commissure attachment areas where commissure portions of the two adjacent leaflets are attached. One of such commissure attachment areas (251) is shown in Fig. 25. Due to shape memory imparted to the frame, the frame can self-expand from a radially collapsed state achieved normally at lower temperatures (generally in an ice-bath), where the metallic material is in martensite phase, to a pre-defined diameter when the restraining force applied for collapsing the valve is removed while the valve is exposed to a higher temperature encountered in a blood stream where the frame metal transforms to austenite phase.

The self-expandable THV is further provided with at least two leaflets (not shown), preferably three leaflets made from animal tissue or synthetic materials. The commissure portions of two adjacent leaflets are attached to the frame 250 at the commissure attachment areas 251 of the frame 250 to form commissures of the valve.

In addition, the prosthetic valve may be provided with at least one of an inner skirt and an outer skirt as described for the balloon expandable prosthetic valve (not shown).

At least one, preferably at least two, eyelet/s, loop/s or retainer/s (252) are provided at the outflow end A of the frame 250 to capture tab/s or paddle/s or fit into receptacles provided in the delivery system for anchoring the prosthetic valve to the catheter (described below).

A person skilled in art is well aware of different designs of the self-expanding prosthetic valve. The frame 250 shown in Fig. 25 is for illustration.

As shown in Fig. 25, the frame 250 of the exemplary self-expanding THV is tubular in shape. The frame 250 includes a proximal end, a distal end and an axis passing across the proximal and distal ends. The frame of the embodiment shown in Fig. 25 has varying diameters across its axial length. However, the frame may have other shapes such as an hourglass shape, tubular shape of uniform diameter etc. One of the commissure attachment areas 251 of the frame 250 is also shown in Fig. 25.

There are two loops (252) provided on the outflow end of the frame in the exemplary embodiment of the aortic prosthetic valve shown in Fig. 25. It may be noted that, in this embodiment, none of the loops (252) is aligned to (i.e. on the same axis as) any of the commissure attachment areas (251) of the frame. To achieve commissural alignment, it is convenient that at least one of the loops (252) should be aligned with one of the commissure attachment areas (251) of the prosthetic valve.

A skilled person will readily understand that this invention can be used for the self-expanding prosthetic valve of any scaffold design.

### Delivery system for self-expanding THV:

As mentioned above, to achieve Commissural Alignment, novel additional features are required to be provided on the delivery system. This section describes these additional features for a delivery system i.e. a catheter for self-expanding THV.

The delivery system of a self-expanding THV consists of a catheter and a loading system. The loading system is used to load the prosthetic valve on the catheter shaft in radially collapsed condition or crimped condition. A person skilled in the art is well familiar of various designs of the delivery system for a self-expanding THV. The method for commissure alignment described herein is applicable to delivery system for self-expanding THV system of any design available in market.

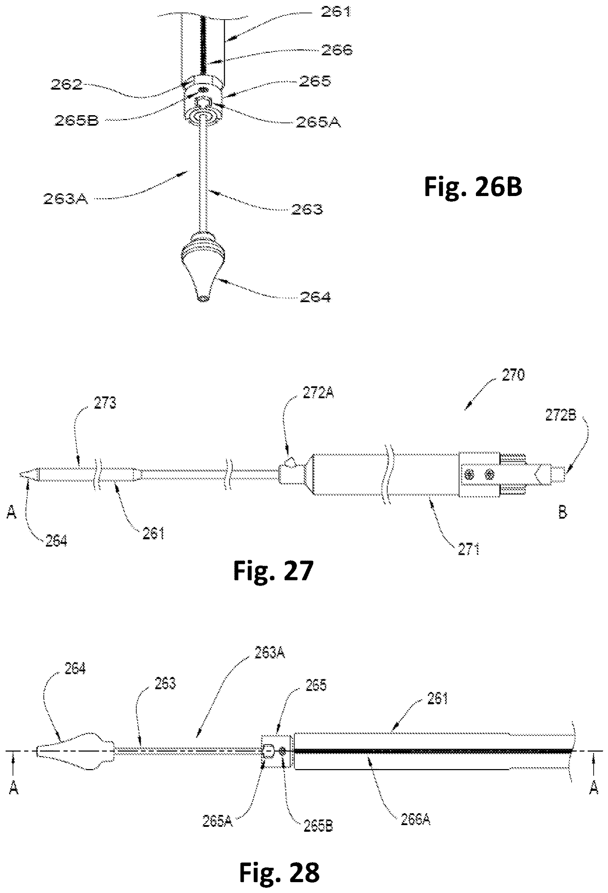

Fig. 26 shows a distal portion of a typical exemplary delivery catheter 260 for a self-expanding prosthesis such as a THV. Fig. 26A shows sectional view and Fig. 26B shows a perspective view of the distal portion of the delivery catheter 260 of Fig. 26 where 'A' designates the distal end and 'B' designates the proximal end.

It may be noted that the distal portion 260 of the delivery catheter shown in Figs. 26, 26A and 26B is exemplary. A skilled person is well aware of delivery catheters of varied designs but with similar operating principles. A conventional delivery catheter for a self-expanding THV generally comprises an elongated outer shaft 261, an intermediate shaft 262 passing coaxially through the outer shaft 261 and an inner lumen 263 passing coaxially through the intermediate shaft 262. An atraumatic tip 264 is generally attached to the distal end of the inner lumen 263. A handle is provided at the proximal end of the delivery catheter (not shown in figures 26, 26A and 26B). The outer shaft 261, the intermediate shaft 262 and the inner lumen 263 (collectively referred to as "the tubes") extend till the proximal end of the catheter and enter inside the handle. The inner lumen 263 acts as a guidewire lumen. The outer shaft 261 is slidable linearly with respect to the intermediate shaft 262 in axial direction. The intermediate shaft 262 and the inner lumen 263 are fixed and are not slidable with respect to each other. The tubes are not rotatable with respect to each other. The mechanism of sliding movement of the outer shaft 261 is normally housed in the proximal handle. The handle may also be provided with other mechanisms as required for the operation of the delivery catheter. As mentioned above, this description is exemplary and general in nature. Delivery catheters of alternate design features are deemed to be covered under the scope of this invention.

Normally, the outer shaft 261 of the catheter is slid over the crimped prosthetic valve to retain it in radially collapsed (crimped) condition. Alternately, a retainer sheath is provided for covering the prosthetic valve to retain it in radially collapsed (crimped) condition.

The self-expanding THV is normally mounted on the distal end of the inner lumen 263 in the area 263A, near and proximal to the distal tip 264, in radially collapsed/crimped condition (prosthetic valve is not shown). The outer shaft 261 is configured to cover the radially collapsed prosthetic valve to retain it in crimped condition. A delivery catheter of another design may have a retainer sheath that covers the self-expanding THV in radially collapsed condition. The prosthetic valve can be expanded by gradually retracting the outer shaft 261 or the retainer sheath in proximal direction to uncover the prosthetic valve allowing it to self-expand. Typically, the handle provided at the proximal end of the catheter houses a mechanism to effect required movement of the outer shaft 261 or the retainer sheath in controlled manner. As mentioned above, the tubes and retainer sheath (if provided) are not rotatable relative to each other.

The delivery system 260 may optionally be provided with a mechanism to flex the distal end portion of the catheter shaft for ease of moving it through aortic arc. The mechanism for flexing may also be housed in the proximal handle.

A hub or holder 265 is generally provided on the intermediate shaft 262. The distal end of the holder 265 of the exemplary embodiment is flush with the distal end of the intermediate shaft 262. As shown in Figs. 26, 26A and 26B, the prosthetic valve is mounted on the inner lumen 263 in radially collapsed (crimped) condition in the space 263A between the proximal end of the tip 264 and the distal end of the holder 265. Proximal end of the prosthetic valve mounted on the inner lumen in radially collapsed condition may abut the distal end of the holder 265. The function of the holder 265 is to retain the position of the prosthetic valve. Generally, as described above, the frame 250 of the prosthetic valve has at least one, or preferably at least two, loop/s or eyelet/s (252) at one of its ends. The holder 265 is provided with corresponding number of tabs/paddles (265A) at its distal end which fit into the loops/eyelets (252) in the frame 250. Alternately, the holder 265 is provided with corresponding number of receptacle areas at its distal end to receive the loop/s or eyelet/s of the crimped prosthetic valve. When the prosthetic valve is mounted on the inner lumen in collapsed condition, the loops/eyelets (252) on the frame 250 are engaged with the tabs/paddles/receptacle areas (265A) on the holder 265. This arrangement restricts the axial and rotational movements of the collapsed THV and retains its position relative to the catheter shafts. Exemplary embodiment of Figs. 26, 26A and 26B shows holder 265 with receptacle area 265A for receiving the loop/s or eyelet/s (252) of the frame 250.

The holder 265 is not fixed rigidly onto the intermediate shaft 262 or the inner lumen 263. It is free to rotate relative to the intermediate shaft and the inner lumen. A locking screw 265B, as shown in Figs. 26, 26A and 26B, is provided on the holder 265 to lock the position of the holder 265 to restrict its rotational and translational motion with respect to the intermediate shaft 262 as well as the inner lumen 263. A skilled person is aware of various other methods of locking and unlocking components like holder onto a shaft like inner lumen. For the exemplary embodiment described, the locking is achieved by tightening the screw 265B. When the screw 265B is loosened, the holder 265 is unlocked and is free to rotate.

The outer diameter of the holder 265 (along with the screw tightened on the inner lumen) is less than the inner diameter of the outer shaft 261 and that of the retainer sheath (if provided) so that the outer shaft 261/retainer sheath can slide over the holder 265.

Fig. 27 shows assembly of the typical delivery catheter 270, distal portion 260 of which is described above and shown in Figs. 26, 26A and 26B. It may be noted that the delivery catheter of Fig. 27 is just exemplary. This invention is deemed to cover the delivery catheters of other designs. The embodiment of Fig. 27 shows the outer shaft 261 covering the prosthetic valve (not shown) in radially collapsed (crimped) condition at the distal end A, proximal to the tip 264. As previously mentioned, the prosthetic valve may alternatively be covered with a retaining sheath instead of the outer shaft. A handle 271 is provided at the proximal end B of the delivery catheter which houses various mechanisms for functioning of the delivery catheter. Points for flushing the shafts/tubes of the delivery system may be provided on the handle. Two exemplary flushing points 272A and 272B are shown in Fig. 27.

The delivery catheter system for a self-expanding THV is normally provided with a loading system. Before procedure, the operator can crimp the prosthetic valve manually over the inner lumen and cover it with the outer shaft or a retainer sheath with the help of such loading system. The loading system generally consists of one or more conically shaped and tubular components which help in gradually reducing the diameter of the prosthetic valve. This operation is generally carried out at a lower temperature, usually in an ice-bath to allow transition of the shape memory alloy to its martensite state and the diameter of the prosthetic valve is reduced without distorting the struts. The prosthetic valve is crimped over the inner lumen 263 at its designated location 263A as described above and covered with the outer shaft 261 or the retainer sheath to retain it in the crimped condition over the area 273. There may be other accessories which further help in the crimping procedure. Each manufacturer of the self-expanding devices such as THV system supply loading system of different design. A skilled person is quite familiar with different loading systems supplied by suppliers of self-expanding THVs.

The self-expanding THV is deployed by gradually retracting the outer shaft 261 or the retaining sheath in proximal direction to uncover the prosthetic valve allowing it to self-expand at human body temperature. As mentioned above, this operation is controlled with the help of the mechanism that is normally provided as a part of the proximal handle 271.

There are several self-expanding prosthetic valves available in the market with delivery catheters and loading system components of different designs. However, the basic method of loading and deployment is similar in all of them. The loading system of all of them has one or more conically shaped and tubular components which play key role in reducing the diameter of the prosthetic valve.

The delivery catheter for self-expanding prosthetic valve may optionally include a flexing mechanism by which the distal portion of the catheter shaft may be flexed for ease of movement through aortic arc.

The same basic principles described previously for a balloon expandable prosthetic valve can be applied to a self-expanding prosthetic valve for achieving commissural alignment. Following novel changes are necessary to achieve commissural alignment for a self-expanding THV. These changes do not alter the design of the system.

The outer shaft 261 of the delivery catheter 270 for the self-expanding THV is provided with single or multiple aligners 266 in the same manner as described previously for the delivery system for a balloon expandable THV. In case of multiple aligners 266, they are spaced apart from each other, preferably equidistant from each other in a single orientation i.e. on the same axis as described previously for delivery system for balloon expandable THV. A convenient way of marking the aligners 266 is such that they follow same axis as the company logo on the proximal handle 271 and continue on the same axis (i.e. retain same orientation) throughout. This is merely a convenient way of marking the aligners 266. It is not necessary to follow the same axis as company logo. Fig. 26 shows an embodiment showing one of the multiple aligners 266 at the distal end 260 of the outer shaft 261. It may be noted that the outer shaft 261 may be alternately provided with a single continuous aligner 266A in place of multiple aligners 266 as shown in Fig. 28 which shows distal end portion of the delivery catheter 270.

The distal end of the single or the distalmost aligner 266/266A on the catheter shaft 261 ends at the distal end of the outer shaft 261. If a retaining sheath is provided, the aligner 266/266A may also be provided on the retaining sheath.

Various alternate ways of providing aligners described previously for the delivery catheter for a balloon expandable THV, such as painting, pasting strips, marking by a laser beam and other methods known in the art, are applicable to delivery catheter for self-expanding THV as well. The colour of the aligners may be in contrast with the colour of the outer shaft to enhance plane visibility. The aligners may also be radiopaque so that they are also visible under fluoroscopy. In case the outer shaft 261 is marked with multiple aligners 266, as mentioned previously for delivery system for a balloon expandable THV, they may be spaced apart from each other in a single orientation i.e. on the same axis preferably equidistant from each other.

As described previously and shown in Fig. 25, the frame 250 of a self-expanding prosthetic valve has loops/eyelets 252 at its outflow end A. To achieve commissural alignment, a convenient way is to axially align at least one of the loops/eyelets 252 on the frame 250 to one of the commissures 251 of the THV. To differentiate this loop/eyelet 252 from the other loops/eyelets, identifying marking/s may be provided on this loop/eyelet. These identifying markings should be easily identifiable visually.

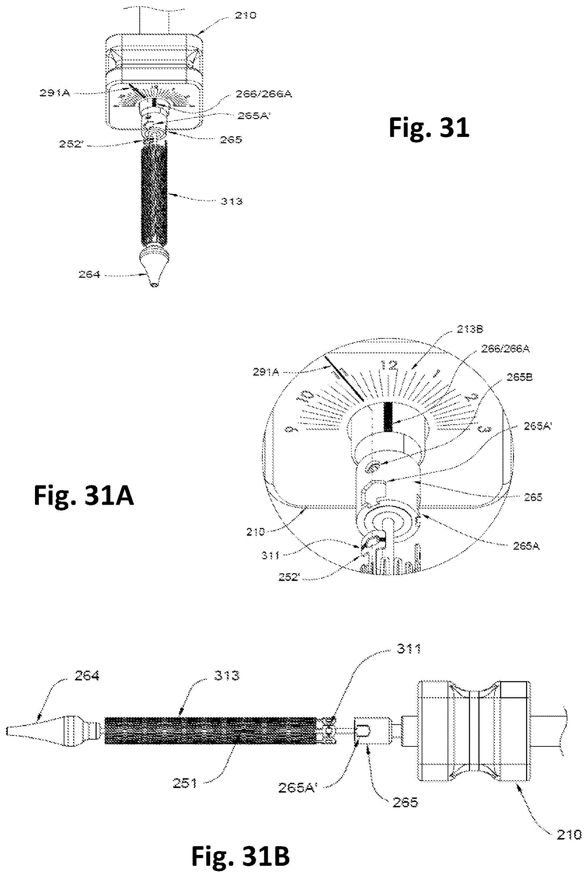

Exemplary identifying markings 311 on a loop/eyelet are shown in Fig. 31A. The sole purpose of providing the identifying markings is to distinguish the loop/eyelet that is axially aligned to one of the commissures 251 of the THV. Hence, other ways of making identifying marking/s on the loop/eyelet can also be used.

In case, at least one of the loops/eyelets 252 on the frame is not axially aligned to one of the commissures 251 of the THV, the method of orienting the crimped valve would be little complex as described below.

A confirmation gauge 210 as described previously for balloon expandable THV system and shown in Figs. 21A-D and 22A/B plays a direct role for self-expanding THV. As described previously, the angle markings 213A-D are provided on at least one side of the confirmation gauge 210. The role of the confirmation gauge 210 is described in the method of crimping below.

### Method of crimping the self-expanding THV

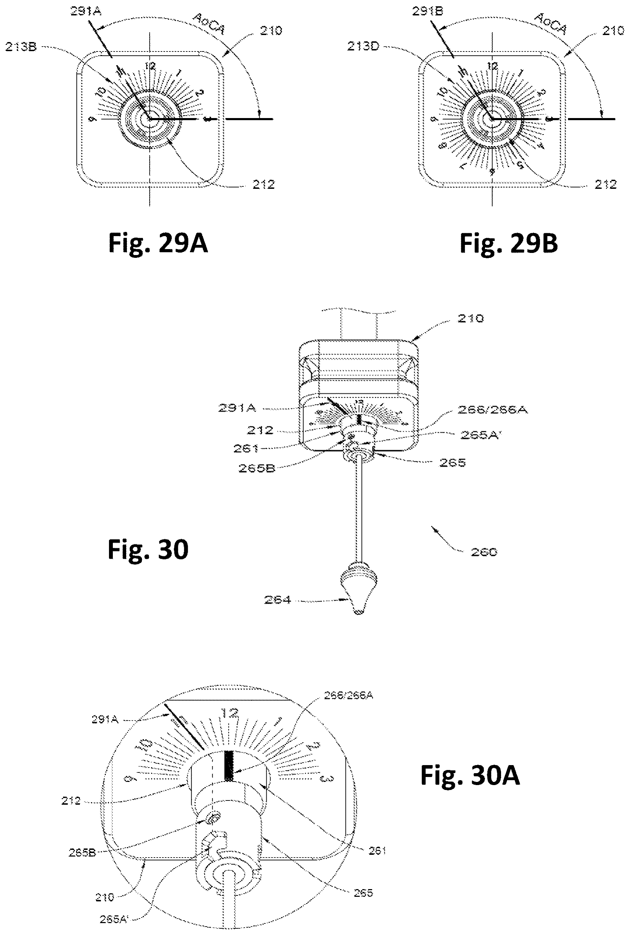

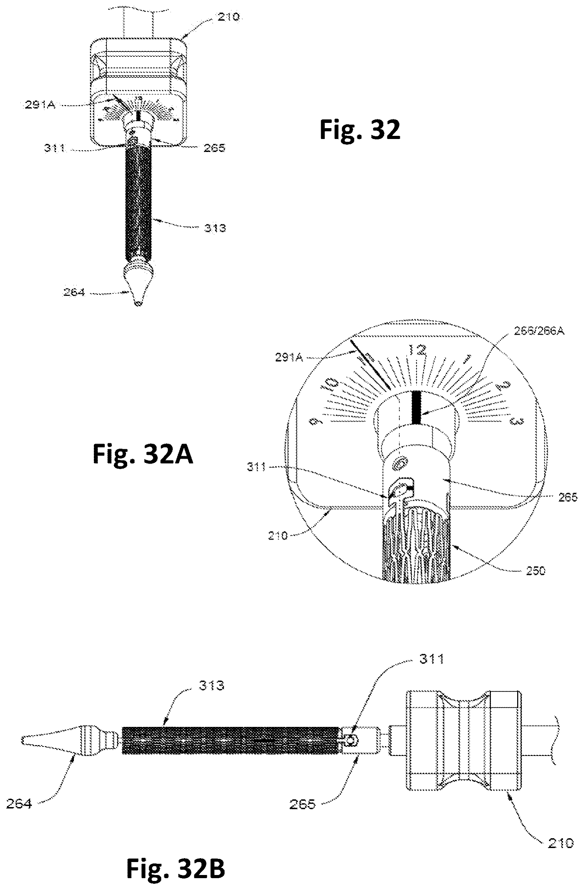

A skilled person is well aware of the crimping procedure of a self-expanding THV. Conventionally, no attention is paid to the orientation of the commissure areas 251 of the frame 250 while mounting the THV on the shaft of a delivery catheter 270. Hence, there is no conscious effort to achieve commissural alignment. The method of crimping a self-expanding THV to achieve commissural alignment is outlined below in step-wise manner.
1. AoCA for the patient is determined in the same manner as described previously. This method is not dependent on the type of the THV (whether balloon expandable or self-expanding). To demonstrate the method, as an example, the measured AoCA is assumed to be an obtuse angle as shown in Fig. 14A/14B.
2. AoCA is identified on the exemplary angle markings 213B or 213D on the confirmation gauge 210. Figs. 29A and 29B show two exemplary embodiments of a confirmation gauge with Clock Angle markings which cover half of the circumference of the central opening 212 in Fig. 29A and full circumference of the central opening 212 in Fig. 29B. AoCA may be identified on it by making a mark 291A/291B on the confirmation gauge as shown in Figs. 29A and 29B respectively. It may be noted that any other method of angle marking (such as in degrees) is equally effective. Also any other method of identifying AoCA on the confirmation gauge is also equally effective.
3. As shown in Fig. 30, the distal end 260 of the delivery catheter 270 is inserted in the central opening 212 of the confirmation gauge 210 from one side of the confirmation gauge 210 such that a portion of the outer shaft 261 and the holder 265 protrude out from the other side of the confirmation gauge 210. This arrangement is shown in Fig. 30A as expanded view for clarity. The holder 265 may be kept in locked position. The catheter is oriented such that the aligner/s 266/266A face upwards as shown in Figs. 30 and 30A.
4. This step is followed when at least one of the loops/eyelets 252 on the frame 250 is axially aligned to one of the commissures 251 of the THV and is provided with the identifying markings 311. The holder 265 is unlocked e.g. by loosening the locking screw 265B on the holder 265. The holder 265 is rotated on the intermediate shaft 262 such that one of the tabs/paddles or receptacle areas on the holder 265A is aligned with AoCA marking 291A on the confirmation gauge 210 as shown in Fig. 30. For clarity, Fig. 30A shows an expanded view of the front surface of the confirmation gauge 210 and the holder 265. The dotted line on these figures shows the alignment of one of the receptacle areas 265A of the holder 265 with AoCA marking 291A on the confirmation gauge 210. This receptacle area is shown as 265A'. In this position, the holder 265 is locked e.g. by tightening the locking screw 265B taking care that the aligner 266/266A on the outer shaft 261 remains oriented upwards at the same time. As tubes cannot rotate relative to each other and the holder 265 is locked in position, the orientation of the catheter tubes and the holder 265 is fixed relative to each other based on AoCA. The holder 265 is kept in locked condition thereafter.
5. The crimping of the prosthetic valve is done using the loading system in such a manner that the loop/eyelet on the frame 250 of the prosthetic valve that has identifying marking/s (one that is aligned to one of the commissures of the prosthetic valve) is aligned with the tab/paddle or the receptacle 265A' on the holder 265 that is aligned to AoCA marking 291A on the confirmation gauge 210. Fig. 31 shows the crimped valve 313 aligned in such a manner. For clarity, only the valve frame is shown in Fig. 31 without leaflets and other parts of the valve. A close up view is shown in Fig. 31A for further clarity. Exemplary identifying markings 311 on the loop/eyelet 252' that is aligned to one of the commissures of the valve are also shown. Fig. 31B shows a side view of the assembly shown in Figs. 31 and 31A. All the time, as shown in Figs. 31 and 31A, the aligner/s 266/266A should face upwards. Figs. 31 and 31A show the exemplary embodiment with confirmation gauge 210 marked with Clock angles 213B and receptacle areas 265A and 265A' to receive loops/eyelets 252 and 252'. The component identification numbers with an apostrophe refer to the component with specific features. 265A refers to receptacle area on the holder 265, while, 265A' refers to one of the receptacle areas which is aligned with AoCA marking 291A on confirmation gauge 210. Similarly, 252 refers to the loops or eyelets on the frame 250 of prosthetic valve, while, 252' refers to the loop/eyelet that is aligned with one of the commissure attachment areas 251 of the prosthetic valve frame 250.
6. The loops/eyelets 252 and 252' are then engaged with the tabs/paddles or the receptacle areas 265A and 265A' on the holder 265 as shown in Fig. 32 and in a close up view in Fig. 32A such that (a) the loop/eyelet of the frame 250 that is aligned with one of the commissures of the valve (with identifying marking/s) 252' is engaged with the receptacle area 265A' of the holder 265 that is aligned with the AoCA marked on the confirmation gauge 210 and (b) the aligner 266/266A on the outer shaft 261 or the retainer sheath (if provided) is facing upward. Fig. 32B shows side view of the arrangement shown in Fig. 32. Figs. 32 and 32A/B show an exemplary embodiment of the delivery catheter where the holder has receptacle areas. In this manner, one of the commissure areas of the THV is axially aligned with AoCA and simultaneously, the aligner/s point upward just as what is described for balloon expandable THV.
7. The outer shaft 261 or the retainer sheath (if provided) of the delivery catheter is then moved over to cover the crimped valve 313 and the holder 265 to retain the crimped valve 313 in this position in radially collapsed condition.
8. Now the system is ready for introducing the catheter into the patient's vasculature using recommended introducer sheath.

In case, any one of the loops/eyelets 252 on the frame 250 is not axially aligned to one of the commissures of the THV, the procedure of crimping becomes a little complex. In this case, the holder 265 is unlocked and rotated on the intermediate shaft 262 such that one of the tabs/paddles or receptacle areas 265A on the holder is oriented such that when the eyelet/loop 252 of the crimped THV is engaged with the tab/paddle or the receptacle 265A on the holder, one of the commissures 251 of the THV is aligned with AoCA marking 291A on the confirmation gauge 210. The fundamental point is to align one of the commissures of the THV with AoCA keeping the aligner/s 266/266A facing upward.

### Method of implantation

The method of implantation for the self-expanding THV mounted on the delivery catheter using crimping method mentioned above, to achieve commissure alignment is described below.
1. Insert the distal end of the delivery catheter that has the prosthetic valve mounted on it in radially collapsed condition into the patient's vasculature through an introducer sheath keeping the aligner/s 266/266A facing upwards. If the aligners follow the axis of the company logo on proximal handle, it is convenient to keep the company logo facing upwards. This orientation should not be changed during the implantation procedure.

Hence, do not torque the delivery system. In case inadvertent torquing happens during insertion of the THV system due to anatomical challenges, the aligners can help the operator to reorient the system to un-torque. However, the system may be flexed if it has the flexing mechanism.
2. Navigate the delivery catheter through the patient's vasculature to the aortic annulus of the patient.
3. Once the prosthetic valve and the balloon have crossed the aortic annulus, one of the commissures of the prosthetic valve is expected to align towards a native commissure. The other commissures of the prosthetic valve would automatically align towards other native commissures.
4. Park the prosthetic valve at the desired target location for deployment.
5. Deploy the prosthetic valve by withdrawing the outer shaft or the retaining sheath proximally to uncover the crimped prosthetic valve, allowing it to self-expand and get deployed at the target location. The expected final deployment with commissural alignment is as shown in Fig. 24.

The skilled person would readily understand that this method is also applicable for different types of bicuspid aortic valve anatomy. In bicuspid anatomy, the skilled person would measure AoCA angle to achieve minimal commissural misalignment using above mentioned technique.

This invention relies on three known factual aspects viz. (a) fluoroscopic view is a mirror image of anatomic/AP view; (b) a THV which is deployed under fluoroscopic guidance with just one commissure aligned towards the mirror image of mid-RCC as per fluoroscopic (and e.g. MSCT) view, will actually deploy anatomically towards the NCC-LCC commissure with minimal mis-alignment and (c) a THV which is deployed under fluoroscopic guidance with just one commissure aligned towards the mirror image of mid-LCC as per fluoroscopic (and e.g. MSCT) view, will actually deploy anatomically towards the RCC-NCC commissure with minimal mis-alignment.

Thus, by using this technique, it is possible to predictably ensure minimal misalignment of native aortic valve commissures to the commissures of the prosthetic valve.

The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

Various embodiments of the present invention are described in the following numbered clauses:
1. A method for implanting a prosthetic valve in a patient's body with minimum misalignment of commissures of the prosthetic valve to the commissures of a native aortic valve, the method comprising:
   a. determining an Angle of Commissural Alignment (AoCA) of a native aortic valve of a patient to be treated;
   b. crimping a prosthetic valve having three commissures on a delivery system having one or more aligners marked on its outer shaft wherein, the one or more aligners follow same axis, the crimping of the prosthetic valve on the delivery system is done using at least one of a crimper or a confirmation gauge that includes one or more angle markings on at least one of its faces, the crimping of the prosthetic valve on the delivery system is performed such that one of the commissures of the prosthetic valve is axially aligned with the AoCA identified on the angle markings on one of the crimper or the confirmation gauge and at the same time, the one or more aligners face upwards; and
   c. implanting the crimped prosthetic valve by maintaining the one or more aligner/s pointing upward during the entire implantation procedure.
2. The method of clause 1 wherein, the method of determining the AoCA consists of:
   a. examining anatomy of a patient's aortic root which houses the native aortic valve and capturing a transverse cross-sectional image of mid-SoV in an imaging software,
   b. drawing horizontal and vertical center-lines on a virtual circle drawn by the software in the image of the mid-SoV and identifying the intersection of these center-lines as "geometric nodule of sinuses" (GNS),
   c. drawing a line through the GNS to a geometric mid-point of any one of Right Coronary Cusp (RCC), Left Coronary Cusp (LCC) or Non-Coronary Cusp (NCC); and
   d. measuring an angle formed between the line drawn at (c) and the horizontal center-line that extends on right hand side of the GNS as the AoCA.
3. The method of clause 2 wherein, the imaging software is multi-slice computed tomography (MSCT).
4. A delivery system for implanting a prosthetic valve, the delivery system comprising:
   a. a proximal end and a distal end, wherein the distal end refers to the end away from the operator,
   b. an elongated outer shaft having a proximal end and a distal end, and
   c. a handle at the proximal end of the outer shaft,
      wherein, the outer shaft is provided with at least one aligner starting at the proximal end of the handle with reference to an axis,
      wherein, the at least one aligner end at or near the distal end of the outer shaft; and
      wherein, the at least one aligner follows the same axis and retains same orientation from the proximal handle till the distal end of the outer shaft.
5. The delivery system of clause 4 wherein, there are multiple aligners spaced apart from each other, wherein, the proximal most aligner starts at the proximal handle and the distal most aligner ends at or near the distal end of the outer shaft.
6. The delivery system of clause 4 wherein, the delivery system is provided with a single aligner in the form of a continuous line extending from the proximal handle to the distal end of the outer shaft.
7. The delivery system of clauses 4 to 6, wherein, the colour of the aligner(s) on the outer shaft of the delivery system is in contrast with the colour of the outer shaft so as to provide improved visibility to the aligners.
8. The delivery system of clauses 4 to 7, wherein, the aligners on the delivery system are radiopaque so as to be visible under fluoroscopy.
9. The delivery system of clauses 4 to 7, wherein, the aligners are painted on the outer shaft of the delivery system.
10. The delivery system of clause 9, wherein the paint used for the aligners is radiopaque.
11. The delivery system of clauses 4 to 7, wherein, the aligners are in the form of strips of a biocompatible material pasted on the outer shaft of the delivery system.
12. The delivery system of clause 11, wherein the strips of the aligners are radiopaque.
13. The delivery system of clauses 4 to 6, wherein, the aligners are marked on the outer shaft by laser beam.
14. An assembly used for implanting a balloon expandable prosthetic aortic valve in a patient's body using the method of implanting the prosthetic aortic valve in accordance with clause 1, to achieve minimum misalignment of commissures of a prosthetic aortic valve to the commissures of a native aortic valve, comprising:
   a. a prosthetic aortic valve which is radially expandable and collapsible and suitable for mounting on a balloon of a delivery system in radially collapsed condition, the prosthetic valve comprising:
      a. a radially expandable and collapsible frame with three commissure attachment areas;
      b. three leaflets attached to the frame at least at the three commissure attachment areas to form commissures of the prosthetic aortic valve;
   b. a delivery system of any of the clauses 4-13 to deliver and deploy the prosthetic valve;
   c. a crimper to radially collapse the prosthetic aortic valve on the balloon of the delivery system wherein,
      a. the crimper is provided with angle markings on at least one of its external surfaces around its central opening.
15. The method of clause 1 wherein, the step of crimping of the prosthetic valve being a balloon expandable prosthetic valve includes:
   a. placing a crimper on a preparation table with an iris opening in an open position,
      wherein the crimper consists of angle markings on at least one of its sides, wherein the angle markings are in the form of at least one of degrees or clock angles and identifying an angle marking that corresponds to AoCA determined as per clause 2;
   b. placing the prosthetic valve at least partially across the iris opening of the crimper such that any one of the commissures of the prosthetic valve is aligned to the identified angle marking on the crimper that corresponds to AoCA,
   c. placing a balloon of the delivery system across the prosthetic valve held inside the iris opening of the crimper, wherein the outer shaft of the delivery system is marked with aligner/s, such that the said aligner/s face upwards,
   d. crimping the prosthetic valve on the balloon of the delivery system either fully or partially maintaining the position of one of the commissures of the prosthetic valve aligned to the identified angle marking on the crimper that corresponds to the AoCA and the aligner/s face upwards during the entire crimping process, and
   e. opening the iris opening of the crimper and removing the balloon with the partially or fully crimped prosthetic valve from the iris opening of the crimper.
16. The method of clause 15 wherein, a line is marked on the angle markings on the crimper identifying the AoCA on the angle markings.
17. The method of clause 15 wherein, the step of crimping the prosthetic valve partially includes:
   a. removing the balloon of the delivery system along with the partially crimped prosthetic valve from the iris opening,
   b. inserting the partially crimped prosthetic valve along with the balloon of the delivery system with aligner/s facing upwards into a central opening of the confirmation gauge,
   c. checking whether any one of the commissures of the prosthetic valve is aligned with the angle marking on the confirmation gauge that corresponds to the AoCA,
   d. if not, adjusting the position of the prosthetic valve by rotating the prosthetic valve on the balloon such that one of the commissures of the prosthetic valve is aligned with the angle marking on the confirmation gauge that corresponds to the AoCA,
   e. removing the prosthetic valve partially crimped on the balloon of the delivery system from the opening of the confirmation gauge,
   f. inserting the balloon of the delivery system along with the prosthetic valve partially crimped on the balloon of the delivery system into the iris opening of the crimper, and
   g. crimping the prosthetic valve on the balloon of the delivery system fully and firmly following the method of clause 15.
18. The method of clause 1 wherein, the method of implanting the crimped prosthetic valve being a balloon expandable prosthetic valve, includes:
   a. inserting an introducer sheath in the vasculature of the patient, preferably into a femoral artery;
   b. inserting a distal end of the delivery system having the balloon with the prosthetic valve crimped on it into the introducer sheath with the aligners on the outer shaft of the delivery catheter facing upwards,
   c. maintaining upward orientation of the aligners while navigating the crimped prosthetic valve through the patient's vasculature to desired deployment site in the aortic annulus;
   d. deploying the prosthetic valve by inflating the balloon of the delivery system maintaining upward orientation of the aligners; and
   e. deflating the balloon of the delivery system after deploying the prosthetic valve; and
   f. withdrawing the delivery system shaft along with the balloon out from the patient's vasculature.
19. The method of clause 18, wherein the inserting the introducer sheath includes inserting the introducer sheath in one of, a carotid artery, sub-clavian artery or axillary artery.
20. The method of clause 18, wherein the inserting the introducer sheath includes inserting the introducer sheath through one of, apex of the heart for trans-apical implantation route or vein and crossing over into aorta using trans-caval implantation route.
21. A confirmation gauge comprising:
   a. a central circular opening which can accommodate the delivery system of clauses 4-13 along with the prosthetic valve partially crimped on it; and
   b. a plurality of angle markings at least on one side of the confirmation gauge, around the central opening.
22. The confirmation gauge of clause 21, wherein, the confirmation gauge has a square structure.
23. The confirmation gauge of clause 21, wherein, the confirmation gauge has a rectangular structure.
24. The confirmation gauge of clause 21, wherein, the confirmation gauge has a circular structure.
25. The confirmation gauge of clause 21, wherein, the angles of the angle markings are expressed as degrees.
26. The confirmation gauge of clause 21, wherein, the angles of the angle markings are expressed as clock angles.
27. The confirmation gauge of clause 21, wherein, the angle markings correspond to the angle markings on the crimper.
28. The confirmation gauge of clause 21, wherein, a line is marked on the angle markings on the confirmation gauge for easy identification of AoCA on the angle markings.
29. An assembly for implanting a self-expandable prosthetic aortic valve in a patient's body using the method of implanting the prosthetic aortic valve in accordance with clause 1 to achieve minimum misalignment of commissures of the prosthetic aortic valve to the commissures of a native aortic valve, comprising:
   a. a prosthetic aortic valve which is radially expandable and collapsible and suitable for mounting on a delivery system; the prosthetic valve comprising:
      i. a proximal end, a distal end and an axis passing across the proximal and distal ends;
      ii. a self-expanding frame which is radially expandable and collapsible, the frame comprises three commissure attachment areas;
      iii. three leaflets attached to the frame at least at the three commissure attachment areas to form commissures of the prosthetic aortic valve;
      iv. at least one loop or eyelet at one of the ends of the frame, wherein, the at least one loop or eyelet is axially aligned to any one of the commissures of the prosthetic valve,
   b. a delivery system of any of the clauses 4-13 to deliver and deploy the prosthetic valve;
   c. a loading system to radially collapse the prosthetic aortic valve on the inner lumen of the delivery system; and
   d. a confirmation gauge of any of the clauses 21-28 to accommodate the delivery system of clauses 4-13 along with the prosthetic valve at least partially crimped on it to orient the commissures of the prosthetic valve with AoCA.
30. The assembly of clause 29, wherein, the at least one loop or eyelet at one of the ends of the self-expanding frame that is aligned to one of the commissures of the prosthetic valve is provided with one or more identifying marks for visual identification.
31. The assembly of clause 29, wherein, the delivery system including a retainer sheath having at least one aligner marked on the retainer sheath.
32. The method of clause 1 wherein, the step of crimping the prosthetic valve being a self-expandable prosthetic valve with one of the commissures of the prosthetic valve aligned to at least one of the loops or eyelets on one of the ends of the frame of the prosthetic valve, includes:
   a. identifying the AoCA, determined according to clause 2, on the angle markings on at least one of the sides of the confirmation gauge, wherein the angle markings are in the form of at least one of degrees or clock angles,
   b. locking a holder on the intermediate shaft of the delivery system using a locking mechanism wherein, the holder is located on the intermediate shaft of the delivery system, and wherein the holder is provided with a plurality of tabs or paddles or receptacle areas,
   c. inserting a distal end of the delivery system in the central opening of the confirmation gauge from one side of the confirmation gauge such that a portion of the outer shaft and the holder protrude out from the other side of the confirmation gauge and the delivery system is oriented such that the aligner/s provided on the outer shaft of the delivery catheter face upwards,
   d. unlocking the holder and rotating it on the intermediate shaft such that one of the tabs/paddles or receptacle areas on the holder is aligned with the identified AoCA marking on the confirmation gauge,
   e. locking the holder on the distal end of the intermediate shaft in this position,
   f. crimping the prosthetic valve on the inner lumen between the distal end of the holder and proximal end of a tip using a loading system in such a manner that the loop/eyelet on the frame of the prosthetic valve that is aligned to one of the commissures of the prosthetic valve is engaged to the tab/paddle or the receptacle on the holder that is aligned to the identified angle marking on the confirmation gauge that corresponding to the AoCA,
   g. moving the outer shaft or the retainer sheath of the delivery system over to cover the crimped valve and the holder to retain the crimped valve in this position in radially collapsed condition.
33. The method of clause 1 wherein, the step of implanting the prosthetic valve being a self-expanding prosthetic valve, includes:
   a. inserting the distal end of the delivery system that has the prosthetic valve mounted on it in radially collapsed condition into the patient's vasculature through an introducer sheath keeping the aligner/s provided on the outer shaft of the delivery catheter facing upward,
   b. navigating the delivery system through patient's vasculature under fluoroscopic guidance till the prosthetic valve and the balloon have crossed the aortic annulus taking care that the aligner/s face upward during the entire implantation procedure,
   c. parking the prosthetic valve at the target implantation location, and
   d. allowing the prosthetic valve to self-expand by withdrawing the outer shaft or the retaining sheath gradually in the proximal direction to uncover the crimped prosthetic valve maintaining the upward orientation of the aligner/s.

## Claims

1. A method for crimping a prosthetic valve on a delivery system for delivering the prosthetic valve:
wherein the prosthetic valve comprises at least two commissures;
wherein the delivery system comprises an outer shaft (161, 261) comprising one or more aligners (166,266) marked thereon wherein the one or more aligners (166, 266) are spaced apart from each other in an axial direction and on the same axis and have the same axial orientation with respect to the outer shaft; and
wherein a crimper (190) or a confirmation gauge (210) includes one or more angle markings (193, 213A - 213D) on at least one of its faces;
wherein the method comprises
identifying an angle marking (193, 213A - 213D) of the one or more angle markings (193, 213A - 213D) corresponding to an Angle of Commissural Alignment (AoCA) of a native aortic valve of a patient to be treated;
crimping the prosthetic valve on the delivery system such that one of the commissures is axially aligned with the identified angle marking (193, 213A-213D), and at the same time, the one or more aligners (166, 266) face upwards with respect to an opening of the crimper (190) or confirmation gauge (21).

2. The method of claim 1 further comprising the step of determining the AoCA of the patient to be treated, optionally wherein the step of determining the AoCA of the patient consists of:
a. capturing a transverse cross-sectional image of the Mid-Sinuses of Valsalva (mid-SoV) in an imaging software;
b. drawing horizontal and vertical center-lines (HCL, VCL) on a virtual circle drawn by the software in the image of the mid-SoV and identifying the intersection of the horizontal and vertical center-lines as "geometric nodule of sinuses" (GNS);
c. drawing a line (L) through the GNS to a geometric mid-point of any one of Right Coronary Cusp (RCC), Left Coronary Cusp (LCC) or Non-Coronary Cusp (NCC); and
d. measuring an angle formed between the line drawn at (c) and the horizontal center-line (HCL) that extends on the right-hand side of the GNS as the AoCA.

3. The method of claim 2 wherein, the imaging software is multi-slice computed tomography (MSCT).

4. The method of claim 1 wherein the prosthetic valve is a balloon expandable prosthetic valve, and the method comprises:
a. placing the crimper (190) on a preparation table, wherein the crimper comprises an iris opening (191A) which is brought into an open position,
b. placing the prosthetic valve at least partially across the open iris opening (191A) of the crimper (190) such that one of the commissures of the prosthetic valve is aligned to the identified angle marking on the crimper (190) that corresponds to the AoCA;
c. placing a balloon (164) of the delivery system across the prosthetic valve held inside the open iris opening (191A) of the crimper (190), wherein the one or more aligners (166) on the outer shaft (161) of the delivery system face upwards with respect to the iris opening (191A);
d. crimping the prosthetic valve on the balloon (164) of the delivery system either fully or partially by closing the iris opening, maintaining the position of one of the commissures of the prosthetic valve aligned to the identified angle marking on the crimper (190) that corresponds to the AoCA, wherein the one or more aligners (166) on the outer shaft (161) of the delivery system face upwards with respect to the iris opening (191A) during this step, and
e. opening the iris opening (191A) of the crimper (190) and removing the balloon (164) with the partially or fully crimped prosthetic valve from the iris opening (191A) of the crimper (190).

5. The method of claim 4, wherein the angle markings (193) on the crimper (190) are in the form of at least one of: degrees or clock angles.

6. The method of claim 4 wherein, a line is marked on the angle markings (193) on the crimper (190) to identify the AoCA of the patient to be treated from the angle markings (193).

7. The method of claim 4 wherein the method further comprises:
a. removing the balloon of the delivery system along with the partially crimped prosthetic valve from the iris opening,
b. inserting the partially crimped prosthetic valve along with the balloon (164) of the delivery system into a central opening (212) of the confirmation gauge (210), wherein the one or more aligners (166) marked on the outer shaft (161) of the delivery system face upwards with respect to the central opening;
c. checking whether any one of the commissures of the prosthetic valve is aligned with the angle marking (213B) on the confirmation gauge (210) that corresponds to the AoCA;
d. if not, adjusting the position of the prosthetic valve by rotating the prosthetic valve on the balloon (164) such that one of the commissures of the prosthetic valve is aligned with the angle marking (213B) on the confirmation gauge (210) that corresponds to the AoCA;
e. removing the balloon (164) of the delivery system along with the prosthetic valve partially crimped on the balloon (164) from the central opening (212) of the confirmation gauge (210);
f. inserting the balloon (164) of the delivery system along with the prosthetic valve partially crimped on the balloon (164) of the delivery system into the iris opening (191A) of the crimper (190), and
g. crimping the prosthetic valve on the balloon (164) of the delivery system fully and firmly following the method of claim 4.

8. The method of claim 1 wherein the prosthetic valve is a self-expandable prosthetic valve comprising a frame (250) having loops or eyelets (252) on one end, where at least one of the commissures of the prosthetic valve is aligned to at least one of the loops or eyelets, wherein the method further comprises:
a. locking a holder (265) located on an intermediate shaft (262) of the delivery system using a locking mechanism wherein, the holder (265) is provided with a plurality of tabs / paddles or receptacle areas (265A);
b. inserting a distal end of the delivery system in a central opening (212) of the confirmation gauge (210) from one side of the confirmation gauge (210) such that a portion of the outer shaft (261) of the delivery system and the holder (265) protrude out from the other side of the confirmation gauge (210) with the one or more aligners (266) provided on the outer shaft (261) of the delivery system facing upwards with respect to the central opening;
c. unlocking the holder (265) and rotating the holder (265) on the intermediate shaft (262) such that one (265A') of the tabs/paddles or receptacle areas (265A) on the holder (265) is aligned with the identified AoCA marking (291A) on the confirmation gauge (210);
d. locking the holder (265) on the distal end of the intermediate shaft (262) in this position,
e. crimping the prosthetic valve on an inner lumen (263) between a distal end of the holder (265) and a proximal end of a tip (264) such that the loop/eyelet (252') on the frame (250) of the prosthetic valve that is aligned to one of the commissures of the prosthetic valve is engaged to the tab/paddle or the receptacle (265A') on the holder (265) that is aligned to the identified angle marking (291A) on the confirmation gauge (210) that corresponds to the AoCA; and
f. moving the outer shaft (261) of the delivery system or a retainer sheath of the delivery system to cover the crimped valve and the holder (265) to retain the crimped valve in this position in radially collapsed condition.

9. The method of claim 1 wherein, the prosthetic valve is a self-expandable prosthetic valve comprising a frame (250) having at least one loop or eyelet (252) provided at one end thereof, wherein the method comprises:
a. locking a holder (265) located on an intermediate shaft (262) of the delivery system using a locking mechanism wherein, the holder (265) is provided with a plurality of tabs / paddles or receptacle areas (265A);
b. inserting a distal end of the delivery system in a central opening (212) of the confirmation gauge (210) from one side of the confirmation gauge (210) such that a portion of the outer shaft (261) of the delivery system and the holder (265) protrude out from the other side of the confirmation gauge (210) with the one or more aligners (266) provided on the outer shaft (261) of the delivery system facing upwards with respect to the central opening;
c. unlocking the holder (265) and rotating the holder (265) on the intermediate shaft (262) to orient one of the tabs/paddles or receptacle areas (265A) on the holder (265) such that when a corresponding eyelet or loop (252) of the prosthetic valve is engaged with the one tab/paddle or receptacle area (265A) of the holder (265), one of commissures of the prosthetic valve is aligned with the identified AoCA marking (291A) on the confirmation gauge (210);
d. locking the holder (265) on the distal end of the intermediate shaft (262) in this position;
e. crimping the prosthetic valve on an inner lumen (263) between a distal end of the holder (265) and a proximal end of a tip (264) such that the at least one loop/eyelet (252) on the frame (250) of the prosthetic valve is engaged with the corresponding tabs/paddles or receptacle areas (265A) of the holder (265) such that one of the commissures of the prosthetic valve is aligned to the identified angle marking (291A) on the confirmation gauge (210) that corresponds to the AoCA; and
f. moving the outer shaft (261) of the delivery system or a retainer sheath of the delivery system to cover the crimped valve and the holder (265) to retain the crimped valve in this position in radially collapsed condition.

10. The method of claim 1, wherein the prosthetic valve has three commissures.
